# FASCICULE DE BREVET EUROPEEN

(11) **EP 3 016 974 B1**
(45) Date de publication et mention de la délivrance du brevet: **11.03.2020**
(21) Numéro de dépôt: 14761690.8
(22) Date de dépôt: 04.07.2014
(51) Int. Cl.: C07K 14/47, C07K 7/08, A61K 39/00

(54) **PEPTIDES IMMUNOGÈNES DE L'ANTIGÈNE TUMORAL CYCLINE B1**
IMMUNOGENE PEPTIDE VON DEM TUMOR-ANTIGEN CYCLIN B1.
IMMUNOGENIC PEPTIDES FROM TUMORAL ANTIGEN CYCLIN B1.

(30) Priorité: 05.07.2013 FR 1356661
(43) Date de publication de la demande: 11.05.2016
(73) Titulaire: Commissariat à l'Énergie Atomique et aux Énergies Alternatives, 75015 Paris (FR)
(72) Inventeur: MAILLERE, Bernard, F-78000 Versailles (FR); CHEVALEYRE, Claire, F-75014 Paris (FR); CASTELLI-GOLFIER,Florence, F-92320 Chatillon (FR); FAVRY, Emmanuel, F-91600 Savigny Sur Orge (FR); MOUHMADI, Anaïs, F-94140 Alfortville (FR)
(74) Mandataire: Plasseraud IP
(86) Numéro de dépôt international: PCT/IB2014/062863
(87) Numéro de publication internationale: WO 2015/001526

(56) Documents cités:
- WO-A2-03/033520
- WO-A2-2010/011994
- US-A1- 2010 291 082
- VELLA LAURA A ET AL: "Healthy individuals have T-cell and antibody responses to the tumor antigen cyclin B1 that when elicited in mice protect from cancer", PROCEEDINGS OF THE NATIONAL ACADEMY OF SCIENCES OF THE UNITED STATES OF AMERICA, vol. 106, no. 33, août 2009 (2009-08), pages 14010-14015, XP002724172, ISSN: 0027-8424 cité dans la demande
- DATABASE Geneseq [Online] 9 décembre 2010 (2010-12-09), "Helical protein secondary structure fragment, SEQ ID 7954.", XP002724173, extrait de EBI accession no. GSP:AYK82740 Database accession no. AYK82740
- GRAZIANO DANIEL F ET AL: "Characterization of T cell responses to cyclin B1 as a tumor antigen", FASEB JOURNAL, vol. 16, no. 4, 20 mars 2002 (2002-03-20), page A668, XP008169260, & ANNUAL MEETING OF THE PROFESSIONAL RESEARCH SCIENTISTS ON EXPERIMENTAL BIOLOGY; NEW ORLEANS, LOUISIANA, USA; APRIL 20-24, 2002 ISSN: 0892-6638
- RIKKE BAEK SORENSEN ET AL: "CD8 T-cell responses against Cyclin B1 in breast cancer patients with tumors overexpresssing p53", CLINICAL CANCER RESEARCH, vol. 15, no. 5, 1 mars 2009 (2009-03-01), pages 1543-1549, XP002724174, cité dans la demande
- SUZUKI H. ET AL: "Tcell-dependent antibody responses against aberrantly expressed cyclin B1 protein in patients with cancer and premalignant disease", CLINICAL CANCER RESEARCH, vol. 11, no. 4, 15 février 2005 (2005-02-15), pages 1521-1526, XP002724175, DOI: 10.1158/1078-0432.ccr-04-0538 cité dans la demande
- KAO HENRY ET AL: "Identification of cyclin B1 as a shared human epithelial tumor-associated antigen recognized by T cells", JOURNAL OF EXPERIMENTAL MEDICINE, vol. 194, no. 9, 5 novembre 2001 (2001-11-05), pages 1313-1323, XP002724176, ISSN: 0022-1007 cité dans la demande
- KAO H ET AL: "IDENTIFICATION OF CYCLIN B1 AS AN EPITHELIAL TUMOR ANTIGEN", FASEB JOURNAL, FED. OF AMERICAN SOC. FOR EXPERIMENTAL BIOLOGY, US, vol. 15, no. 5, 8 mars 2001 (2001-03-08), page A1206, XP009051575, ISSN: 0892-6638
- MIN YU ET AL: "Aberrant cyclin B1 expression in human tumors and cell lines", FASEB JOURNAL, vol. 15, no. 5, 8 mars 2001 (2001-03-08), page A1206, XP002724177, & ANNUAL MEETING OF THE FEDERATION OF AMERICAN SOCIETIES FOR EXPERIMENTAL BIOLOGY ON EXPERIMENTAL BIOL; ORLANDO, FLORIDA, USA; MARCH 31-APRIL 04, 2001 ISSN: 0892-6638
- ANDERSEN RIKKE SICK ET AL: "Identification of a cyclin B1-derived CTL epitope eliciting spontaneous responses in both cancer patients and healthy donors", CANCER IMMUNOLOGY IMMUNOTHERAPY, vol. 60, no. 2, février 2011 (2011-02), pages 227-234, XP002724178, ISSN: 0340-7004
- KLEIN-GONZALEZ NELA ET AL: "Cyclins against cancer: a novel family of tumor antigens?", IMMUNOTHERAPY, vol. 2, no. 5, septembre 2010 (2010-09), pages 595-597, XP002724179, ISSN: 1750-743X

## Description

L'invention est relative à des peptides immunogènes issus de l'antigène tumoral cycline B1 qui sont capables de se lier à de nombreuses molécules HLA de classe II (HLA II) et d'induire chez l'homme une activation spécifique des lymphocytes T CD4+ chez des sujets présentant des molécules HLA II variées. L'invention est également relative à l'utilisation de tels peptides comme vaccin anticancéreux.

La différenciation des cellules en cellules tumorales est le résultat d'une dérégulation génétique qui favorise leur prolifération et leur expansion dans l'organisme. Cette dérégulation se traduit par l'expression de protéines normalement peu ou non exprimées dans les cellules normales ou exprimées de manière transitoire. Parmi ces protéines, certaines sont exprimées en quantité suffisante pour être reconnues par le système immunitaire et constituent des antigènes tumoraux. La vaccination anti-tumorale utilise comme cible ces antigènes tumoraux afin de pouvoir induire une réponse immunitaire spécifique des tumeurs qui épargne les cellules saines. Ces antigènes sont répartis en plusieurs catégories, en fonction de leur mode d'expression.

Depuis la découverte de lymphocytes T CD8⁺ cytotoxiques (CTL) spécifiques des tumeurs, un effort important a été mené afin d'induire l'activation de ces cellules. De nombreux travaux portent sur la localisation des peptides que reconnaissent les lymphocytes T CD4⁺ étant donné qu'il est maintenant reconnu que l'induction de CTL est dépendante de l'activation de ces cellules (Assudani et al., Cancer Immunol. Immunother., 2007, 56, 70-80), que les lymphocytes T CD4⁺ exercent un contrôle des tumeurs via des mécanismes indépendants des CTL probablement via l'activation de macrophages et qu'ils peuvent également être cytotoxiques. Les lymphocytes T CD4⁺ reconnaissent les peptides (dénommés épitopes T CD4⁺) de tumeurs que leur présentent les molécules HLA de classe II. La reconnaissance peut avoir lieu de manière directe (c'est-à-dire que la tumeur présente elle-même ces peptides aux lymphocytes T) mais il est vraisemblable que la voie principale d'activation passe par l'intermédiaire des cellules dendritiques. Ces cellules qui possèdent un nombre élevé de molécules HLA de classe II à leur surface et sont capables de capturer des débris cellulaires des tumeurs sont en effet, les principales cellules présentatrices de l'antigène, aptes à recruter *in vivo* des lymphocytes T naïfs.

Il existe trois types de molécules HLA de classe II: HLA-DR, HLA-DQ et HLA-DP. La molécule HLA-DR codée par le gène DRB1 est la plus exprimée et la plus étudiée. Elle semble participer le plus à la réponse des lymphocytes T CD4⁺ qui sont en effet le plus souvent restreints à HLA-DRB1. De nombreux individus possèdent d'autres locus codant pour les molécules HLA-DR et appelés DRB3, DRB4 et DRB5. Il existe de nombreux variants des molécules HLA II. Ainsi, 1285 allèles HLA-DRB1 différents, codant pour 959 protéines différentes, sont présents dans le monde. Toutefois, les allèles ne sont pas répartis de manière uniforme dans le monde. Par exemple, en Europe et aux USA, 7 molécules HLA-DR codées par HLA-DRB1 (HLA-DRB1*01:01, 15:01, 03:01, 04:01, 11:01, 13:01, 07:01), les allèles HLA-DRB3*01:01, -DRB4*01:01 et- DRB5*01:01 et les molécules HLA-DP4 codée par les allèles (DPB1*0401 et DPB1*0402) sont prépondérantes et couvrent l'essentiel de la population (Tableau I).

**Tableau I : Fréquence phénotypique/allélique* des principales molécules HLA de classe II**

| Fréquence des individus (%) | | | | | | | |
|---|---|---|---|---|---|---|---|
| **Allèles** | **France** | **Allemagne** | **USA Caucasien** | **USA noir** | **Sénégal** | **Indes** | **Japon** |
| DRB1*0101 | 17,7/**9,3** | 13,0/**6,7** | 14,1/**7,3** | 3,8/1,9 | 1,2/0,6 | 7,5/3,8 | 9,6/4,9 |
| DRB1*0401 | 10,9/**5,6** | 15,5/**8,1** | 13,0/**6,7** | 3,0/1,5 | 0,0/0,0 | 1,8/0,9 | 0,0/0,0 |
| DRB1*1101 | 17,6/**9,2** | 17,6/**9,2** | 8,6/4,4 | 15,7/**8,2** | 17,7/**9,3** | 1,8/0,9 | 4,0/2 |
| DRB1*0701 | 26,0/**14,0** | 23,1/**12,3** | 26,7/**14,4** | 18,6/**9,8** | 15,0/**7,8** | 24,3/**13** | 1,2/0,6 |
| DRB1*0301 | 20,6/**10,9** | 17,9/**9,4** | 18,1/**9,5** | 13,5/**7** | 19,4/**10,2** | 10,3/**5,3** | 0,8/0,4 |
| DRB1*1301 | 11,6/**6,0** | 8,8/4,5 | 9,9/**5,1** | 8,2/4,2 | 9,2/4,7 | 12,2/**6,3** | 1,4/0,7 |
| DRB1*1501 | 15,4/**8,0** | 15,0/**7,8** | 19,5/**10,3** | 16,5/**8,6** | 0,0/0,0 | 22,7/**12,1** | 17,4/**9,1** |
| TOTAL | 86,3/63,0 | 82,4/58,0 | 82,1/57,7 | 65,4/41,2 | 54,6/32,6 | 66,7/42,3 | 32,3/17,7 |
| | | | | | | | |
| DRB5*0101 | 15,2/**7,9** | 9,0/4,6 | 4,7/2,4 | 19,7/**10,4** | 0,0/0,0 | 0,0/0,0 | 10,9/**5,6** |
| DRB3*0101 | 17,6/**9,2** | 18,6/**9,8** | 19,7/**10,4** | 27,9/**15,1** | 13,3/**6,9** | 9,6/4,9 | 12,6/**6,5** |
| DRB4*0101 | 48,2/**28,0** | 37,7/**21,1** | 35,7/**19,8** | 30,3/**16,5** | 13,3/**6,9** | 43,4/**24,8** | 49,4/**28,9** |
| TOTAL | 69,9/45,1 | 58,4/**35,5** | 54,6/32,6 | 66,4/42,0 | 25,7/13,8 | 50,6/29,7 | 65,2/41,0 |
| | | | | | | | |
| DPB1*0401 | 64,0/**40,0** | 61,7/**38,1** | 43,9/**25,1** | 20,8/**11** | 9,4/4,8 | nd | 9,2/4,7 |
| DPB1*0402 | 20,8/**11,0** | 28,4/**15,4** | 23,6/**12,6** | 17,2/**9** | 44,5/**25,5** | nd | 60,1/**36,8** |
| TOTAL | 76,0/51,0 | 78,4/53,5 | 61,2/37,7 | 36,0/20 | 51,4/30,3 | nd | 65,8/41,5 |

| | | | | | | | |
|---|---|---|---|---|---|---|---|
| *Les molécules HLA II prépondérantes (fréquence allélique > 5 % sont indiquées en gras) | | | | | | | |

Toutes les molécules HLA de classe II adoptent une structure tridimensionnelle très similaire. Leur site de fixation des peptides est constitué par un plancher en feuillet béta sur lequel reposent deux hélices alpha. Le site est ouvert aux deux extrémités et possède cinq poches de spécificité (P1, P4, P6, P7 et P9) correspondant aux positions des résidus du peptide qu'elles accommodent (Stern et al., Nature, 1994, 368, 215-221). Les peptides qui se lient aux molécules HLA de classe II ont généralement une taille comprise entre 11 et 25 acides aminés. Comme les différences de séquences entre les molécules HLA de classe II sont essentiellement localisées dans les poches du site de fixation des peptides, les épitopes T CD4⁺ varient d'un individu à l'autre en fonction de leurs molécules HLA de classe II.

De manière à être efficace, la réponse en lymphocytes T CD4⁺ spécifiques d'antigènes tumoraux doit avoir une amplitude (ou intensité) suffisante en mobilisant un nombre important de lymphocytes et doit avoir lieu chez une majorité d'individus.

De récents travaux montrent que l'amplitude de la réponse en lymphocytes T CD4⁺ contre un antigène (ou réponse T CD4⁺ spécifique) est dépendante de la taille du répertoire en lymphocytes T CD4⁺ spécifiques de cet antigène qui circulent dans l'organisme (Moon et al., Immunity, 2007, 27, 203-213). Cette taille du répertoire est très variable d'un antigène à l'autre. Elle résulte d'une part de l'ensemble des réarrangements du Tcr qui peuvent reconnaître l'antigène et de la sélection thymique. Il est possible de quantifier le répertoire spécifique d'un antigène par différentes méthodes qui s'appuient, soit sur l'emploi de tétramères de HLA de classe II (Jenkins et al., J. Immunol., 2012, 188, 4135-4140), soit sur l'amplification des lymphocytes T CD4⁺ par des stimulations non spécifiques (Geiger, et al., J. Exp. Med., 2009, 206, 1525-1534) ou spécifiques (Delluc et al., Blood, 2010, 116, 4542-4545; Delluc et al., Faseb J., 2011, 25, 2040-2048 ; Demande Internationale WO 2010/076413). Les cellules T CD4⁺ spécifiques étant présentes à une fréquence très faible dans le sang des donneurs, les lymphocytes T CD4⁺ spécifiques sont amplifiés par des cycles de stimulation antigénique ; la spécificité des cellules T CD4⁺ pour les peptides est évaluée après amplification. Ces tests ont une valeur prédictive de l'intensité de la réponse des lymphocytes T CD4⁺ spécifiques chez l'homme et permettent d'identifier les protéines ou les peptides ayant le répertoire en lymphocytes T CD4⁺ spécifiques le plus important et donc capables de stimuler les réponses en lymphocytes T CD4⁺ spécifiques les plus fortes. En outre, réalisés sur un ensemble de donneurs ayant des molécules HLA de classe II variées, ces tests permettent de mettre en évidence les protéines, les peptides ou les combinaisons de peptides ayant une fréquence de répondeurs élevée. Ainsi, le test d'amplification des lymphocytes T CD4⁺ par des stimulations spécifiques a permis de distinguer des anticorps thérapeutiques immunogéniques de ceux qui ne le sont pas (Delluc et al., Faseb J., 2011, 25, 2040-2048) et de rendre compte de l'immunogénicité de l'érythropoïétine recombinante (Delluc et al., Blood, 2010, 116, 4542-4545).

Compte tenu du polymorphisme des molécules HLA de classe II, la capacité d'un antigène à stimuler des lymphocytes T CD4⁺ spécifiques chez une majorité d'individus dépend de sa capacité à être présenté par de nombreuses molécules HLA de classe II. Cette capacité de présentation dépend de l'affinité des séquences peptidiques présentes dans l'antigène pour les molécules HLA de classe II, les épitopes T CD4⁺ étant en effet principalement trouvés dans les peptides de forte affinité. La spécificité de liaison des molécules HLA de classe II étant très variable d'une molécule HLA de classe II à une autre, ces peptides varient d'un individu à un autre. Toutefois, certains peptides peuvent se lier à plusieurs molécules par une zone commune d'interaction avec les molécules HLA de classe II. Ils peuvent également abriter plusieurs zones d'interaction avec les molécules HLA de classe II. L'évaluation de la fréquence de répondeurs à un antigène ou à des peptides issus de cet antigène nécessite la prise en compte du polymorphisme des molécules HLA de classe II. Une première évaluation peut être effectuée en mesurant l'affinité de peptides issus de l'antigène pour les molécules HLA de classe II les plus fréquentes. Cette affinité peut notamment être mesurée par des tests de liaison spécifiques des molécules HLA-DR (Brevet US 6,649,166) et HLA-DP4 (Demande Internationale WO 03/040299). Une évaluation plus précise est effectuée par un test d'amplification des lymphocytes T par des stimulations spécifiques, réalisé à partir de suffisamment d'individus possédant des molécules HLA II variées pour couvrir l'ensemble des molécules HLA de classe II les plus fréquentes (Demande Internationale WO 2010/076413).

La cycline B1 (CCNB1) est une protéine de 433 acides aminés et de 48kDa, impliquée dans la régulation du cycle cellulaire et plus particulièrement dans la transition de la phase G2 vers la phase M. La cycline B1 humaine correspond à la séquence UniProt P14635 ou SEQ ID NO : 1. Dans une cellule saine, elle s'associe durant la phase G2 du cycle cellulaire avec la Kinase Dépendante des Cyclines 1 (cdk1 = Cyclin Dependent Kinase 1 aussi appelée cdc2) pour former le Facteur Promoteur de la Phase M (MPF = M-Phase Promoting Factor). L'activation de ce complexe lors de la transition G2/M provoque sa translocation dans le noyau, où il contribue à la fragmentation de l'enveloppe nucléaire, à l'assemblage du fuseau mitotique, à la condensation des chromosomes et à la ségrégation des chromatides. La CCNB1 est ensuite rapidement dégradée par ubiquitination et destruction par le protéasome, ce qui permet l'inactivation de cdc2 et la sortie de la mitose. L'expression de la CCNB1 dans les cellules saines est donc transitoire. La synthèse de la CCNB1 est initiée à la fin de la phase S, puis augmente progressivement pendant la phase G2 pour atteindre un pic au niveau de la transition G2/M, avant d'être stoppée. La CCNB1 est maintenue dans le cytoplasme durant toute la phase G2 avant de devenir nucléaire au début de la mitose (transition G2/M).

CCNB1 est surexprimée dans de nombreux cancers incluant des cancers du sein (Ohta et al., Breast Cancer, 1997, 4, 17-24), du colon (Wang et al., J. Cancer Res. Clin. Oncol., 1997, 123, 124-127), de la prostate (Mashal et al., Cancer Res., 1996, 56, 4159-4163), de l'œsophage (Murakami et al., Virchows Archiv : An International Journal of Pathology, 1999, 434, 153-158), de l'estomac (Yasuda et al., J. Cancer Res. Clin. Oncol., 2002, 128, 412-416), des poumons (Soria et al., Cancer Res., 2000, 60, 4000-4004) et des cancers ORL (Hoffmann et al., Anticancer research, 2011, 31, 3151-3157). Dans les cellules tumorales, la cycline B1 est retrouvée aussi bien dans le noyau que dans le cytoplasme. Cette expression anormale contribue à la croissance des tumeurs (Keyomarsi et al., Proc. Natl. Acad. Sci. U S A, 1993, 90, 1112-1116) comme le montre des expériences d'inhibition de son expression (Yuan et al., Oncogene, 2004, 23, 5843-5852). Cette surexpression semble due à la perte de fonction de la protéine p53 (Yu et al., Mol. Immunol., 2002, 38, 981-987).La cycline B1 représente une cible de choix pour le développement d'un vaccin anticancéreux étant donné qu'elle est fréquemment exprimée dans des cancers variés et indispensable à la croissance tumorale mais exprimée de façon transitoire dans les cellules saines, ce qui est favorable à une faible induction de tolérance.

Plusieurs études ont montré que la cycline B1 (CCNB1) peut être la cible d'une réponse immunitaire. Des peptides issus de la Cycline B1, élués des molécules HLA de classe I extraites de cellules tumorales de différentes origines (Kao et al., J. Exp. Med., 2001, 194, 1313-1323), sont capables d'induire une réponse en CTLs spécifiques (Tableau II; Kao et al., J. Exp. Med., 2001, 194, 1313-1323; Sorensen et al., Clin. Cancer Res., 2009, 15, 1543-1549; Andersen et al., Cancer Immunol. Immunother., 2001, 60, 227-234; Demande Internationale WO 03/033520).

**Tableau II : Epitopes T de CCNB1 publiés (SEQ ID NO : 96 à 109)**

| **Peptides** | **Sequences** | **Restriction** | **Références** |
|---|---|---|---|
| **Epitopes T CD8⁺** | | | |
| P1 | AGYLMELCV | HLA-A2 | Kao *et al*., précité |
| P2 | AGYLMELCM | HLA-A2 | |
| P3 | AGYLMELCF | HLA-A2 | |
| P4 | AGYLMELCC | HLA-A2 | WO 03/033520 |
| P5 | AGYLMELCMA | HLA-A2 | |
| P6 | AGYLMELCFA | HLA-A2 | |
| CB9 (323-331) | AKYLMELTM | HLA-A2 | |
| CB10 (323-332) | AKYLMELTML | HLA-A2 | |
| | | | |
| CB9L2 | ALYLMELTM | HLA-A2 | Sorensen *et al*, précité |
| CB9M2 | AMYLMELTM | HLA-A2 | |
| | | | |
| CB204 (204-212) | ILIDWLVQV | HLA-A2 | Andersen *et al*., précité |

| **Epitopes T CD4⁺** | | | |
|---|---|---|---|
| | | | |
| 215-229 | KFRLLQETMYMTVSI | Inconnu | |
| 219-233 | LQETMYMTVSIIDRF | Inconnu | (Vella et al., Proc. Natl. Acad. Sci. U S A, 2009, 106, 14010-14015). |
| 223-234 | MYMTVSIIDRFM | Inconnu | Demande US 2011/0280897 |

Une réponse humorale spontanée dirigée contre la CCNB1 et *a priori* dépendante des lymphocytes CD4⁺ a été observée chez des patients atteints de cancer (Suzuki et al., Clin. Cancer. Res., 2005, 11, 1521-1526). Des tests de stimulation de lymphocytes T CD4⁺ avec des peptides chevauchants de CCNB1 (12-15 acides aminés) ont montré que seuls trois peptides (CCNB1 215-229, 219-233 et 223-234) couvrant la région 215 à 233 de la protéine étaient capables d'activer des lymphocytes T CD4⁺ et T CD8⁺ chez plusieurs individus sains (Vella et al., Proc. Natl. Acad. Sci. U S A, 2009, 106, 14010-14015 et demande WO 2010/011994).

Toutefois, l'efficacité réelle de ce peptide n'a pas été démontrée étant donné que la fréquence de répondeurs a été déterminée sur des individus d'haplotype HLA inconnu, c'est-à-dire sans prendre en compte le polymorphisme des molécules HLA II. En outre, l'intensité de la réponse T CD4+ à ces peptides n'est pas connue étant donné que seule une analyse qualitative de la réponse T CD4+ à ces peptides été effectuée.

Pour mettre au point des vaccins efficaces contre le cancer, il existe donc un réel besoin de disposer de nouveaux épitopes T CD4⁺ de la cycline B1 capables d'induire une réponse T CD4⁺ efficace, c'est-à-dire d'intensité élevée et chez de nombreux individus.

Les inventeurs ont identifié un ensemble d'épitopes T CD4⁺ immunodominants *in vitro* de la cycline B1 humaine qui sont différents des trois épitopes T CD4⁺ de la région 215-233 précédemment décrits (Tableau IV). Ces épitopes T CD4⁺ immunodominants *in vitro* de la cycline B1 participent à la réponse T CD4⁺ dirigée contre la cycline B1, dans la mesure où ils sont reconnus spécifiquement par des lymphocytes T CD4⁺ générés par stimulation avec la protéine cycline B1. Contrairement aux épitopes T CD4⁺ de l'art antérieur dont la restriction pour les molécules HLA II est inconnue, ces épitopes T CD4⁺ immunodominants *in vitro* sont reconnus chez des individus portant des molécules HLA II variées comprenant toute les molécules HLA-DRB1 les plus fréquentes dans la population caucasienne (HLA-DR1, -DR3, -DR4, DR7, -DR11, -DR13 et -DR15 ; Tableaux I et IV). Les inventeurs ont également identifié des peptides ayant une large spécificité de liaison aux molécules HLA II les plus fréquentes dans la population caucasienne (Tableau VI).

De façon remarquable, les peptides immunodominants *in vitro* incluent les peptides les plus performants, c'est-à-dire capables d'induire la réponse T CD4⁺ spécifique la plus efficace (intensité moyenne de la réponse *in vitro* supérieure à 2,8 % et pouvant atteindre 6,1 % et fréquence de répondeurs *in vitro* supérieure à 65 % et pouvant atteindre 85 % ; Tableau IX), dans une population d'individus portant des molécules HLA II variées, comprenant toutes les molécules HLA-DRB1 les plus fréquentes dans la population caucasienne, couvrant à elles seules 80 % des individus de la population caucasienne.

De tels peptides représentent des candidats particulièrement prometteurs pour le développement d'un vaccin anticancéreux, du fait qu'ils sont issus de la cycline B1 et qu'ils sont capables d'induire une réponse T CD4⁺ spécifique de forte amplitude *in vitro* chez de nombreux individus.

L'invention est telle que définie dans les revendications.

En conséquence, la présente invention a pour objet un peptide isolé, consistant essentiellement en une séquence d'au plus 30 acides aminés, de préférence de 15 à 25 acides aminés, issue de la séquence de la cycline B1 humaine de SEQ ID NO : 1 et comprenant au moins un épitope T CD4⁺ immunodominant *in vitro* de la cycline B1 humaine, ladite séquence d'acides aminés étant sélectionnée dans le groupe constitué par :
a) les séquences d'au plus 30 acides aminés consécutifs (15, 16, 17, 18, 19, 20, 21, 22, 23, 24, 25, 26, 27, 28, 29 ou 30) de la séquence de la cycline B1 humaine de SEQ ID NO : 1, de préférence de 15 à 25 (15, 16, 17, 18, 19, 20, 21, 22, 23, 24, 25) acides aminés consécutifs de ladite séquence, comprenant l'une des séquences SEQ ID NO : 10, 57, 58, 61, 76, 83, 84, 87, 88 et 91, et
b) les séquences de 15 à 25 acides aminés présentant au moins présentant au moins 85 % d'identité, de préférence au moins 90 %, de manière préférée au moins 95% d'identité avec une séquence en a),
à l'exclusion de la séquence de 15 acides aminés constituée par les résidus 279 à 293 de ladite séquence SEQ ID NO : 1, et
ledit peptide de séquence en a) et b) étant capable de stimuler une réponse en lymphocytes T CD4⁺ humains spécifiques telle que l'intensité moyenne de la réponse *in vitro* en lymphocytes T CD4⁺ humains spécifiques dudit peptide est d'au moins 2,8 % dans un groupe d'individus humains exprimant des molécules HLA II variées incluant les molécules HLA II prépondérantes dans la population dont sont issus lesdits individus, incluant au moins les molécules HLA-DR1, HLA-DR3, HLA-DR4, HLA-DR7, HLA-DR11, HLA-DR13 et HLA-DR15, et la fréquence de répondeurs *in vitro* audit peptide est d'au moins 65 % dans ledit groupe d'individus humains.

### Définitions

- On entend par « cycline B1 » ou « CCNB1 », une protéine cycline B1 issue de n'importe quel mammifère ; de préférence, il s'agit de la protéine cycline B1 humaine. La cycline B1 humaine correspond à la séquence en acides aminés UniProt P14635 ou SEQ ID NO : 1.
- On entend par « épitope T CD4⁺ », un peptide de 11 à 25 acides aminés qui lie au moins une molécule HLA II, de préférence une molécule HLA II prépondérante telle que présentée au Tableau I, et est reconnu par des lymphocytes T CD4⁺ spécifiques chez les individus portant cette molécule HLA II; le peptide comprend une séquence de 9 acides aminés incluant les résidus d'ancrage aux molécules HLA II, flanquée d'au moins 1 acide aminé, de préférence au moins 2 ou 3 acides aminés à chacune de ses extrémités.
- On entend par « épitope T CD4⁺ immunodominant *in vitro* de la cycline B1 » un épitope T CD4⁺ de la cycline B1 qui est reconnu par des lymphocytes T CD4⁺ humains préalablement stimulés *in vitro* par au moins deux cycles successifs, de préférence 3 ou 4 cycles successifs, de stimulation par des cellules présentatrices de l'antigène, autologues, présentant la protéine cycline B1, notamment des cellules dendritiques autologues chargées avec la protéine cycline B1.
- On entend par « molécule HLA II prépondérante », une molécule HLA II dont la fréquence allélique est supérieure à 5 % dans la population de référence.
- On entend par « cancer », un cancer associé à la surexpression de la protéine cycline B1 par des cellules tumorales tel que de façon non-limitative : les cancers du sein, du colon, de la prostate, de l'œsophage, de l'estomac, des poumons et les cancers ORL.
- On entend par « fréquence de répondeurs *in vitro* à un peptide selon la présente invention », le pourcentage d'individus pour lesquels des lignées de lymphocytes T CD4⁺ spécifiques dudit peptide ont été obtenues par rapport à l'ensemble des individus testés.

La fréquence de répondeurs peut être déterminée selon la méthode décrite dans la Demande WO 2010/076413.
- On entend par « intensité de la réponse *in vitro* à un peptide selon la présente invention », l'intensité de la réponse *in vitro* en lymphocytes T CD4⁺ humains spécifiques dudit peptide, c'est-à-dire le pourcentage de puits de culture contenant des lymphocytes T CD4⁺ spécifiques dudit peptide par rapport à l'ensemble des puits mis en culture. L'intensité moyenne de la réponse à un peptide selon l'invention correspond à la moyenne sur l'ensemble des donneurs des intensités calculées pour chaque donneur.

L'intensité de la réponse peut être déterminée selon la méthode décrite dans la Demande WO 2010/076413.
- Le pourcentage d'identité d'une séquence d'acide aminé est défini par le pourcentage de résidus d'acides aminés dans une séquence à comparer qui sont identiques à une séquence de référence après alignement des séquences, en introduisant des espaces si nécessaire, de façon à obtenir une identité de séquence maximale. Le pourcentage d'identité est ensuite déterminé selon la formule suivante : Pourcentage d'identité = 100 x [1- (C/R)], tel que C est le nombre de différences entre la séquence de référence et la séquence à comparer sur toute la longueur de la séquence de référence, (i) chaque acide aminé dans la séquence de référence qui n'a pas d'acide aminé correspondant aligné dans la séquence à comparer, (ii) chaque espace dans la séquence de référence et (iii) chaque acide aminé aligné dans la séquence de référence qui est différent d'un acide aminé dans la séquence à comparer constitue une différence, et R est le nombre d'acides aminés dans la séquence de référence sur toute la longueur de l'alignement avec la séquence à comparer (c'est à dire toute la longueur de la séquence de référence), chaque espace généré dans la séquence de référence étant compté comme un acide aminé. L'alignement de séquences en vue de déterminer le pourcentage d'identité d'une séquence peut être réalisé de différentes façons connues de l'homme du métier, par exemple en utilisant des logiciels publics disponibles comme BLAST (Altschul et al., J. Mol. Biol., 1990, 215, 403-). Ce logiciel est de préférence utilisé avec des paramètres par défaut.

L'invention englobe des peptides recombinants ou synthétiques tels que définis ci-dessus. L'activité de liaison des peptides de l'invention vis-à-vis des molécules HLA II est mesurée par un test de liaison spécifique des molécules HLA II, classique, tel qu'un test en compétition avec révélation immuno-enzymatique tel que *décrit* dans le Brevet américain US 6,649,166 et la Demande Internationale PCT WO 03/040299, respectivement pour les molécules HLA-DR et HLA-DP4. La capacité des peptides de l'invention d'induire un réponse en lymphocytes T CD4⁺ spécifiques à partir de précurseurs présents chez des individus naïfs, de stimuler de telles cellules spécifiques chez des individus atteints d'un cancer associé à la surexpression de la cycline B1, la spécificité des lymphocytes T CD4⁺ induits, vis-à-vis des peptides ou de la protéine cycline B1, ainsi que la capacité des peptides de l'invention d'être reconnus par des lymphocytes T CD4⁺ spécifiques, sont évaluées selon les techniques standards connues de l'Homme du métier comme par exemple : un test de prolifération cellulaire, un test ELISPOT (dosage des cellules productrices de cytokines) ou un test de dosage de cytokines intracellulaires, spécifiques d'une cytokine telle que IFN-γ, IL-2, IL-4 ou IL-10.

Les acides aminés sont désignés à l'aide du code à une lettre. Les positions des peptides issus de la cycline B1 sont indiquées en référence à la séquence humaine (SEQ ID NO: 1).

Les molécules HLA II prépondérantes, HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 et HLA-DP402 sont avantageusement codées respectivement par les allèles HLA DRB1*0101, DRB1*0301, DRB1*0401, DRB1*0701, DRB1*1101, DRB1*1301, DRB1*1501, DRB3*0101, DRB4*0104, DRB5*0101, DP*0401 et DP*0402.

Selon une disposition avantageuse des modes de réalisation précédents, la fréquence de répondeurs *in vitro* audit peptide est d'au moins 75 %, dans un groupe d'individus humains exprimant des molécules HLA II variées incluant les molécules HLA II prépondérantes dans la population dont sont issus lesdits individus, incluant au moins les molécules HLA-DR1, HLA-DR3, HLA-DR4, HLA-DR7, HLA-DR11, HLA-DR13, HLA-DR15, et l'intensité moyenne de la réponse *in vitro* en lymphocytes T CD4⁺ humains spécifiques dudit peptide est d'au moins 2,8 %, dans ledit groupe d'individus humains.

L'invention englobe les peptides variants naturels ou synthétiques obtenus par mutation (insertion, délétion, substitution) d'un ou plusieurs acides aminés dans la séquence de la cycline B1, dès lors que ledit peptide dérivé de la séquence de la cycline B1 humaine de SEQ ID NO : 1 conserve ses propriétés d'épitope T CD4⁺ immunodominant *in vitro* de la cycline B1 et est capable de lier des molécules HLA II, notamment des molécules HLA II prépondérantes et de stimuler une réponse en lymphocytes T CD4⁺ humains spécifiques de la cycline B1 avec une intensité de réponse et une fréquence de répondeurs conformes à la présente invention, c'est-à-dire qu'ils sont reconnus par des lymphocytes T CD4⁺ spécifiques de la séquence sauvage ou d'un variant naturel de la cycline B1. Les variants naturels résultent notamment du polymorphisme de la cycline B1. En outre, d'autres variants peuvent être aisément construits étant donné que les résidus d'acides aminés impliqués dans la liaison aux molécules HLA- DR et HLA-DP4 (résidus d'ancrage) et l'effet des modifications de ces résidus sur la liaison aux molécules HLA-DR et HLA-DP4 sont connus de l'Homme du métier ; la Demande Internationale PCT WO 03/040299 enseigne notamment que pour lier HLA-DP4, le résidu en P6 doit être aromatique ou hydrophobe ou consister en un résidu de cystéine (C), et au moins l'un des résidus P1, P9 est tel que P1 est aromatique ou hydrophobe et/ou P9 est aromatique ou hydrophobe ou consiste en un résidu C, D, Q, S, T ou E, alors que le résidu en P4 peut être n'importe quel résidu d'acide aminé. Le Brevet américain US 6,649,166 décrit une méthode générale permettant de déterminer les résidus d'ancrage aux molécules HLA DR (P1, P4, P6, P7 et P9) et la nature des mutations de ces résidus permettant de modifier l'affinité pour les molécules HLA DR. Des motifs de liaison aux molécules HLA DR sont décrits notamment dans Sturnolio et al., Nat. Biotech, 1999, 17, 533-534 et Rammensee et al., Immunogenetics, 1995, 41, 178-228. Le variant peut également comprendre la substitution d'un ou plusieurs résidus de cystéine de la cycline B1 par un autre acide aminé, par exemple une sérine.

De préférence, ledit peptide consiste en une séquence sélectionnée dans le groupe constitué par les séquences SEQ ID NO : 10, 57, 58, 61, 76, 83, 84, 87, 88 et 91.

Ledit peptide comprend avantageusement plusieurs épitopes T CD4⁺ de la protéine cycline B1.

La présente invention a également pour objet une composition immunogène ou vaccinale, caractérisée en ce qu'elle comprend au moins un peptide tel que défini ci-dessus ou un mélange de peptides (peptides isolés ou polypeptide multi-épitopique comprenant moins de 100 acides aminés consécutifs de la cycline B1), éventuellement modifiés (lipopeptide, protéine de fusion), et un véhicule pharmaceutiquement acceptable, une substance porteuse ou un adjuvant.

Le mélange de peptides inclus dans la composition immunogène ou vaccinale selon l'invention est un mélange de peptides isolés ou associés entre eux sous la forme d'un polypeptide multi-épitopique comprenant moins de 100 acides aminés consécutifs de la cycline B1 humaine de SEQ ID NO : 1, ledit mélange comprenant : (i) au moins un premier peptide comprenant au moins un épitope T CD4⁺ immunodominant *in vitro* de la cycline B1 selon l'invention et (ii) au moins un second peptide comprenant au moins un épitope T CD4⁺ différent dudit épitope immunodominant, et/ou un épitope T CD8⁺, et/ou un épitope B, notamment des épitopes T CD4⁺ et/ou T CD8⁺ de la cycline B1 et/ou d'autres antigènes tumoraux tels que notamment MAGE, NY-ESO-1, la Survivine et la Midkine.

Les épitopes T CD4⁺ ou T CD8⁺ issus d'antigènes tumoraux sont notamment décrits sur le site http://www.cancerimmunity.org/peptidedatabase/tumorspecific.htm et dans les Demandes Internationales PCT WO 2007/036638 et WO 2009/153463.

Parmi les épitopes pouvant être incorporés dans le mélange de l'invention, on peut citer notamment :
- les épitopes T CD8⁺ de la cycline B1 tels que définis ci-dessus (Tableau II),
- les épitopes T CD8⁺ de MAGE tels que décrits dans le Brevet US 6,063,900 et la Demande PCT WO 2004/052917,
- les épitopes T CD4⁺ de MAGE tels que MAGE-A3 267-282 restreint à DR1 (Demande Internationale PCT WO 02/095051) ; MAGE-A3 149-160 restreint à DR4 et DR7 (Kobayashi et al., Cancer Research, 2001, 61, 4773-4778) ; MAGE-A3 191-205 et 281-295 restreints à DR11 (Consogno et al., Blood, 2003, 101, 1038-1044 ; Manici et al., J. Exp. Med., 1999, 189, 871-876) et MAGE-A3 121-134 restreint à DR13 (Brevet US 6,716,809) ; MAGE-A1 281-292 restreint à DR15 (Demande Internationale PCT WO 00/78806) ; MAGE-A6 102-116, 121-144, 140-170, 145-160, 150-165 et 246-263 restreints à DR4 (Tatsumi et al., Clinical Cancer Research, 2003, 9, 947-954) MAGE-A1 281-292 restreint à DR15 (Demande Internationale PCT WO 00/78806) ; MAGE-A6 102-116, 121-144, 140-170, 145-160, 150-165 et 246-263 restreints à DR4 (Tatsumi et al., Clinical Cancer Research, 2003, 9, 947-954) et les épitopes MAGE restreints à HLA-DP4 tels que décrits dans la Demande Internationale PCT WO 2007/026078,
- un épitope T CD8⁺ de la survivine choisi parmi : survivine 96-104 (LTLGEFLKL, SEQ ID NO : 92) ou 95-104 (ELTLGEFLKL, SEQ ID NO :93), survivine-2B 80-88 (AYACNTSTL, SEQ ID NO :94) et les peptides tels que décrits dans le Tableau I de Bachinsky et al., Cancer Immun., 2005, 5, 6-,
- un épitope T CD4⁺ de la survivine tel que décrit dans la Demande Internationale PCT WO 2007/036638 et notamment le peptide 19-33, 90-104 ou 93-107,
- un épitope T CD4⁺ et/ou T CD8⁺ de la Midkine tel que décrit dans la Demande Internationale PCT WO 2009/153463,
- un épitope T CD4⁺ universel naturel ou synthétique tel que le peptide de la toxine tétanique TT 830-846 (O'SULLIVAN et al., J. Immunol., 1991, 147, 2663-2669), le peptide de l'hémagglutinine du virus de la grippe HA 307-319 (O'Sullivan *et al*., précité), le peptide PADRE (KXVAAWTLKAA, SEQ ID NO :95; Alexander et al., Immunity, 1994, 1, 751-761) et des peptides issus des antigènes de *Plasmodium falciparum* tels que le peptide CS.T3 (Sinigaglia et al., Nature, 1988, 336, 778-780) et les peptides CSP, SSP2, LSA-1 et EXP-1 (Doolan et al., J. Immunol., 2000, 165, 1123-1137),
- un épitope B constitué par un sucre (Alexander *et al*., précité), ledit épitope B étant de préférence sous la forme d'un glycopeptide, et
- un épitope B de la cycline B1 reconnu spécifiquement par des anticorps dirigés contre ledit antigène tumoral.

Selon un premier mode de réalisation avantageux de ladite composition, le second peptide est un peptide comprenant un épitope T CD4⁺ de la cycline B1, capable de se lier à au moins 6 molécules HLA II prépondérantes différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 et HLA-DP402, sélectionné dans le groupe constitué par :
a) les séquences d'au plus 30 (15, 16, 17, 18, 19, 20, 21, 22, 23, 24, 25, 26, 27, 28, 29 ou 30) acides aminés consécutifs de la séquence de la cycline B1 humaine de SEQ ID NO : 1, de préférence de 15 à 25 (15, 16, 17, 18, 19, 20, 21, 22, 23, 24, 25) acides aminés consécutifs de ladite séquence, comprenant l'une des séquences SEQ ID NO : 33, 39, 40, 41, 42, 43, 44, 45, 48, 49, 50, 54, 60, 69, 73, 74, 75, 85, 86 et 90, et
b) les séquences de 15 à 25 (15, 16, 17, 18, 19, 20, 21, 22, 23, 24, 25) acides aminés, présentant au moins 85 % d'identité, de préférence au moins ou 90 %, de manière préférée au moins 95% d'identité avec une séquence en a).

De préférence, le dit peptide capable de se lier à au moins 6 molécules HLA II prépondérantes différentes, consiste en une séquence sélectionnée dans le groupe constitué par : SEQ ID NO : 33, 39, 40, 41, 42, 43, 44, 45, 48, 49, 50, 54, 60, 69, 73, 74, 75, 85, 86 et 90.

Alternativement, ledit second peptide est un peptide comprenant un épitope T CD4⁺ de la cycline B1, capable de se lier à 3 à 5 molécules HLA II prépondérantes différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 et HLA-DP402, sélectionné dans le groupe constitué par :
a) les séquences d'au plus 30 (15, 16, 17, 18, 19, 20, 21, 22, 23, 24, 25, 26, 27, 28, 29 ou 30) acides aminés consécutifs de la séquence de la cycline B1 humaine de SEQ ID NO : 1, de préférence de 15 à 25 (15, 16, 17, 18, 19, 20, 21, 22, 23, 24, 25) acides aminés consécutifs de ladite séquence, comprenant l'une des séquences SEQ ID NO : 25, 29, 32, 34, 36, 37, 38, 47, 53, 55, 56, 59, 66, 72 et 78, et
b) les séquences de 15 à 25 (15, 16, 17, 18, 19, 20, 21, 22, 23, 24, 25) acides aminés, présentant au moins 85 % d'identité, de préférence au moins 90 %, de manière préférée au moins 95% d'identité avec une séquence en a).

De préférence, le dit peptide capable de se lier à 3 à 5 molécules HLA II prépondérantes différentes, consiste en une séquence sélectionnée dans le groupe constitué par : SEQ ID NO : 25, 29, 32, 34, 36, 37, 38, 47, 53, 55, 56, 59, 66, 72 et 78.

La combinaison d'épitope(s) T CD4⁺ de la cycline B1 telle que définie ci-dessus permet avantageusement d'améliorer la réponse immunitaire anti-tumorale en élargissant la couverture d'individus répondeurs et en intensifiant l'amplitude de la réponse immunitaire.

Le polypeptide multiépitopique comprend la concaténation des peptides comprenant les différents épitopes. Conformément à l'invention, les séquences des différents peptides sont reliées entre elles par une liaison peptidique ou séparées par des séquences exogènes. Le dérivé multiépitopique comprend moins de 50 acides aminés consécutifs de la cycline B1, de préférence moins de 30. Le dérivé peptidique multiépitopique présente une longueur de 20 à 200 acides aminés, de préférence de 30 à 100 acides aminés, de manière préférée d'environ 50 acides aminés.

Le peptide ou polypeptide modifié est issu du peptide ou polypeptide précédents par l'introduction de toute modification au niveau de résidu(s) d'acide aminé, de la liaison peptidique ou des extrémités des peptides, dès lors que ledit peptide ou polypeptide modifié est capable de se lier aux molécules HLA II et d'activer des lymphocytes T CD4⁺ humains spécifiques avec une intensité de réponse et une fréquence de répondeurs conformes à la présente l'invention. Cette ou ces modification(s), de préférence une ou des modification(s) chimique(s), qui sont introduites dans les peptides par les méthodes classiques connues de l'Homme du métier, incluent de façon non-limitative l'une au moins des modifications chimiques suivantes : l'acétylation du résidu d'acide aminé N-terminal et/ou l'amidation du résidu d'acide aminé C-terminal (Maillère et al., Molecular Immunology, 1195, 32, 1377-1385), la substitution d'un acide aminé par un acide aminé non-protéinogénique (acide aminé D ou analogue d'acide aminé) ; l'addition de groupement chimique (lipide, oligo ou polysaccharide) au niveau d'une fonction réactive, notamment de la chaîne latérale R ; la modification de la liaison peptidique (-CO-NH-), notamment par une liaison du type rétro ou rétro-inverso (-NH-CO-) ou une liaison différente de la liaison peptidique ; la cyclisation ; la fusion de la séquence dudit peptide avec celle d'un peptide (épitope d'intérêt pour la vaccination ; étiquette utile pour la purification du peptide, notamment sous forme clivable par une protéase) ; la fusion de la séquence dudit peptide avec celle d'une protéine, notamment une chaîne α d'une molécule HLA I ou HLA II, une chaîne β d'une molécule HLA II ou le domaine extracellulaire de ladite chaîne ou bien encore une séquence de ciblage dans l'endosome dérivée notamment de la chaîne invariable Ii ou de la protéine LAMP-1 ; le couplage à une molécule appropriée, notamment un marqueur, par exemple un fluorochrome ou la biotine. Ces modifications sont destinées en particulier à augmenter la stabilité et plus particulièrement la résistance à la protéolyse, ainsi que la solubilité, l'immunogénicité ou à faciliter la purification ou la détection, soit du peptide selon l'invention, soit de cellules CD4⁺ et/ou CD8⁺ spécifiques dudit peptide.

Au sens de la présente invention on entend par « dérivé peptidique » d'un peptide selon l'invention, un peptide modifié ou un polypeptide multi-épitopique modifié ou non-modifié.

Selon un mode de réalisation avantageux de ladite composition, ledit peptide ou polypeptide modifié comprend au moins une modification chimique sélectionnée dans le groupe constitué par : l'acétylation du résidu d'acide aminé N-terminal et/ou l'amidation du résidu d'acide aminé C-terminal, la substitution d'un acide aminé par un acide aminé non-protéogénique, l'addition d'un groupement lipide, oligosaccharide ou polysaccharide au niveau d'une fonction réactive de la chaîne latérale d'un acide aminé, une ou plusieurs N6-acetyl-lysine(s), phosphoserine(s) et/ou phosphothreonine(s), la modification de la liaison peptidique par une liaison du type rétro ou rétro-inverso et la fusion de la séquence d'acides aminés dudit peptide ou polypeptide avec la séquence d'acides aminés d'une protéine ou d'un polypeptide hétérologues d'intérêt. De préférence, la N-acetyl-lysine est en position 73, la ou les phosphoserine(s) sont en position 126, 128, 133 et/ou 147 et la phosphothreonine est en position 321 de la de la séquence SEQ ID NO : 1.

Selon un autre mode de réalisation avantageux de ladite composition, ledit peptide ou polypeptide modifié comprend l'acétylation de son résidu d'acide aminé N-terminal et/ou l'amidation de son résidu d'acide aminé C-terminal.

Selon un autre mode de réalisation avantageux de ladite composition, ledit peptide ou polypeptide modifié comprend avantageusement une étiquette fusionnée à l'une de ses extrémités, pour la purification ou la détection dudit peptide/polypeptide. L'étiquette, notamment une séquence polyhistidine ou un épitope B d'un antigène, est de préférence séparée de la séquence du peptide/polypeptide par un site de clivage d'une protéase de façon à isoler la séquence du peptide/polypeptide, à partir de la fusion.

Selon un autre mode de réalisation avantageux de ladite composition, ledit peptide ou polypeptide modifié est un lipopeptide ou un lipopolypeptide comprenant, respectivement un peptide et un polypeptide tels que définis ci-dessus.

Ledit lipopeptide/polypeptide est notamment obtenu par addition d'un lipide sur une fonction α-aminée ou sur une fonction réactive de la chaîne latérale d'un acide aminé dudit peptide ou polypeptide; il peut comprendre une ou plusieurs chaînes dérivées d'acides gras en C₄₋₂₀, éventuellement ramifiées ou insaturées (acide palmitique, acide oléique, acide linoléique, acide linolénique, acide 2-amino hexadécanoïque, pimélautide, trimétauxide) ou un dérivé d'un stéroïde. La partie lipidique préférée est notamment représentée par un groupe N^{α}-acétyl-lysine N^{ε}(palmitoyl), également dénommé Ac-K(Pam).

Selon encore un autre mode de réalisation avantageux de ladite composition, ledit peptide ou polypeptide modifié est une protéine de fusion comprenant ledit peptide ou polypeptide fusionné avec une protéine hétérologue (différente de la cycline B1) ou un fragment polypeptidique hétérologue (fragment d'une protéine différente de la cycline B1 ou fragment de la cycline B1 dont la séquence n'est pas directement adjacente à la séquence dudit peptide ou polypeptide dans la séquence de ladite cycline B1).

Le peptide ou le fragment polypeptide peuvent être fusionnés avec l'extrémité NH₂ ou COOH de ladite protéine ou dudit fragment polypeptidique hétérologue ou insérés dans la séquence de ladite protéine ou dudit fragment.

Selon une disposition avantageuse de ladite composition, ladite protéine de fusion est constituée par un peptide ou un polypeptide tels que définis ci-dessus, fusionné avec une séquence de ciblage dans l'endosome, dérivée de préférence d'une chaîne invariable humaine Ii ou de la protéine LAMP-1. Les séquences de ciblage dans l'endosome et leur utilisation pour le ciblage d'antigènes dans l'endosome sont notamment décrites dans Sanderson et al. (Proc. Nat. Acad. Sci. USA, 1995, 92, 7217-7222), Wu et al. (Proc. Nat. Acad. Sci. USA, 1995, 92, 11671-11675) et Thompson et al. (J. Virol., 1998, 72, 2246-2252).

Selon une autre disposition avantageuse de ladite composition, ladite protéine de fusion est constituée par un peptide ou un polypeptide tels que défini ci-dessus, fusionné avec l'une des chaînes d'une molécule HLA, de préférence la chaîne béta d'une molécule HLA II ou la chaîne alpha d'une molécule HLA I, ou bien avec un fragment de celle-ci correspondant à une molécule HLA soluble, notamment un fragment correspondant au domaine extracellulaire précédé du peptide signal homologue ou d'un peptide signal hétérologue. Ledit peptide est avantageusement inséré entre le peptide signal et l'extrémité NH₂ du domaine extracellulaire de la chaîne α ou β, comme décrit pour la molécule HLA-DR (Kolzin et al., PNAS, 2000, 97, 291-296).

Selon encore une autre disposition avantageuse de ladite composition, ladite protéine de fusion est constituée par un peptide ou un polypeptide tels que définis ci-dessus, fusionnés avec une protéine facilitant leur purification ou leur détection, connue de l'Homme du métier comme notamment la Glutathione S-Transférase (GST) et les protéines fluorescentes (GFP et dérivées). Dans ce cas, la séquence du peptide ou du polypeptide est de préférence séparée du reste de la protéine par un site de clivage d'une protéase, pour faciliter la purification dudit peptide ou polypeptide dudit polypeptide.

La présente invention a également pour objet un polynucléotide isolé codant pour un peptide, un polypeptide ou une protéine de fusion tels que définis ci-dessus.

Conformément à l'invention, la séquence dudit polynucléotide est celle de l'ADNc codant pour ledit peptide ou polypeptide ou ladite protéine de fusion. Ladite séquence peut avantageusement être modifiée de façon à ce que l'usage des codons soit optimal chez l'hôte dans lequel elle est exprimée.

La présente invention a également pour objet un vecteur recombinant comprenant ledit polynucléotide.

Au sens de la présente invention, on entend par vecteur une molécule d'acide nucléique capable de transporter un autre acide nucléique à laquelle elle est associée. Un type de vecteur qui peut être utilisé dans la présente invention inclut, de façon non-limitative, une molécule d'ADN ou d'ARN linéaire ou circulaire constituée d'acides nucléiques chromosomiques, non-chromosomiques, synthétiques ou semi-synthétiques, tel que notamment un vecteur viral, un plasmide ou un vecteur à ARN.

De nombreux vecteurs dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de cette séquence sous forme extrachromosomique, ou bien intégration dans le matériel chromosomique de l'hôte), ainsi que de la nature de la cellule hôte. Par exemple, on peut utiliser des acides nucléiques nus (ADN ou ARN) ou des vecteurs viraux tels que les adénovirus, les rétrovirus, les lentivirus et les AAV dans lesquels a été insérée préalablement la séquence d'intérêt ; on peut également associer ladite séquence (isolée ou insérée dans un vecteur plasmidique) avec une substance lui permettant de franchir la membrane des cellules hôtes, telle qu'un transporteur comme un nanotransporteur ou une préparation de liposomes, ou de polymères cationiques, ou bien l'introduire dans ladite cellule hôte en utilisant des méthodes physiques telles que l'électroporation ou la microinjection. En outre, on peut avantageusement combiner ces méthodes, par exemple en utilisant l'électroporation associée à des liposomes.

De préférence, ledit vecteur est un vecteur d'expression comprenant tous les éléments nécessaires à l'expression du peptide, polypeptide, protéine de fusion tels que définis ci-dessus. Par exemple, ledit vecteur comprend une cassette d'expression incluant au moins un polynucléotide tel que défini ci-dessus, sous le contrôle de séquences régulatrices de la transcription et éventuellement de la traduction appropriées (promoteur, activateur, intron, codon d'initiation (ATG), codon stop, signal de polyadénylation, site d'épissage).

La présente description divulgue une cellule hôte procaryote ou eucaryote modifiée, comprenant un peptide, polypeptide, une protéine de fusion, un polynucléotide ou un vecteur tels que définis ci-dessus ; la cellule pouvant être modifiée de façon stable ou transitoire. La cellule est notamment une cellule présentatrice d'antigène telle qu'une cellule dendritique.

La composition vaccinale selon l'invention comprend un véhicule pharmaceutiquement acceptable, une substance porteuse et/ou un adjuvant.

Les véhicules pharmaceutiquement acceptables, les substances porteuses et les adjuvants sont ceux classiquement utilisés.

Les adjuvants sont avantageusement choisis dans le groupe constitué par : les émulsions huileuses, les substances minérales, les extraits bactériens, les oligonucléotides contenant des CpG, la saponine, l'hydroxyde d'alumine, le monophosphoryl -lipide A et le squalène.

Les substances porteuses sont avantageusement sélectionnées dans le groupe constitué par : les liposomes unilamellaires ou multilamellaires, les ISCOMS, les virosomes, les pseudoparticules virales, les micelles de saponine, les microsphères solides de nature saccharidique (poly(lactide-co-glycolide)) ou aurifère, et les nano-particules.

La composition vaccinale comprend une dose efficace de peptide ou mélange de peptides permettant d'obtenir un effet prophylactique/thérapeutique sur le cancer associé à une surexpression tumorale de la cycline B1 tel que défini ci-dessus. Cette dose est déterminée et ajustée en fonction de facteurs tels que l'age, le sexe et le poids du sujet. La composition vaccinale est généralement administrée selon les protocoles usuels de vaccination, à des doses et pendant une durée suffisante pour induire une réponse cellulaire dirigée contre la protéine cycline B1. L'administration peut être sous-cutanée, intramusculaire, intraveineuse, intradermique, intrapéritonéale, orale, sublinguale, rectale, vaginale, intranasale, par inhalation ou par application transdermique.

La composition se présente sous une forme galénique adaptée à une administration choisie : solution stérile injectable, poudre, comprimés, gélules, suspension, sirop, suppositoires, qui sont préparés selon les protocoles standards.

Selon un mode de réalisation avantageux de ladite composition, elle comprend au moins un épitope T CD4⁺ et un épitope T CD8⁺ de la cycline B1, sous forme d'un mélange de peptides, de préférence un polypeptide multi-épitopique, tels que définis ci-dessus.

Les peptides selon la présente invention et les produits dérivés (mélange de peptides, notamment polypeptide multi-épitopique ; protéine de fusion, lipopeptide) peuvent être utilisés en immunothérapie dans le traitement des tumeurs surexprimant la cycline B1. Lesdits peptides ou produits dérivés sont utilisés, soit comme vaccin, soit en thérapie cellulaire, ou bien encore par une combinaison des deux approches.

La thérapie cellulaire comprend, la préparation de cellules présentatrices d'antigènes (cellules dendritiques) par un protocole classique comprenant l'isolement de cellules mononucléées du sang périphérique (PBMC) d'un patient à traiter et la mise en culture des cellules dendritiques en présence de peptide(s) ou de dérivé(s) peptidique(s) tels que définis ci-dessus. Dans une seconde étape les cellules présentatrices d'antigène chargées avec le peptide ou son dérivé sont réinjectées au patient.

La présente invention a également pour objet l'utilisation d'un peptide ou d'un mélange de peptides tels que définis ci-dessus, pour la préparation d'un médicament (vaccin prophylactique ou curatif) destiné à l'immunothérapie prophylactique ou curative d'un cancer associé à la surexpression de la cycline B1.

La présente description divulgue l'utilisation d'au moins un peptide tel que défini ci-dessus pour la préparation d'un réactif d'immunomonitoring de la réponse cellulaire contre la cycline B1, destiné à l'évaluation du pronostic ou au suivi du traitement d'un cancer (chirurgie, radiothérapie, chimiothérapie, immunothérapie). En particulier, ledit réactif comprend un peptide ou une protéine de fusion tels que définis ci-dessus, par exemple marqué et/ou complexé à une molécule HLA, sous la forme de complexes multimériques HLA/peptide comme des tétramères de complexes HLA/peptide, marqués.

La présente description divulgue une méthode *in vitro,* d'immunomonitoring de la réponse cellulaire contre la cycline B1 chez un individu présentant un cancer, caractérisée en ce qu'elle comprend :
- la mise en contact d'un échantillon biologique dudit individu avec un peptide comprenant au moins un épitope T CD4⁺ immunodominant *in vitro* de la cycline B1 ou un dérivé peptidique dudit peptide tels que définis ci-dessus, et
- la détection de lymphocytes T CD4⁺ spécifiques de la cycline B1, par tout moyen approprié.

La méthode d'immunomonitoring permet de suivre l'évolution de la réponse T CD4⁺ dirigée contre la cycline B1 au cours d'un cancer ou bien d'un traitement antitumoral, notamment une immunothérapie antitumorale ; les lymphocytes T CD4⁺ spécifiques de la cycline B1 peuvent être de type TH1 (sécrétion d'IFN-γ), TH2 (sécrétion d'IL-4) ou T régulateur (sécrétion d'IL-10 ou de TGF-β) ; il est attendu que la réponse T de type TH1 est signe d'une évolution favorable pour le patient alors que la réponse T régulatrice est signe d'une évolution défavorable pour le patient. La détection est effectuée à partir d'un échantillon biologique contenant des cellules T CD4⁺, notamment un échantillon de cellules mononucléées isolées à partir d'un prélèvement de sang périphérique (PBMCs). Cette méthode est mise en œuvre selon les techniques classiques connues de l'Homme du métier, telles que décrites notamment dans la Demande Internationale WO 2009/153463 (page 23, ligne 20 à page 26, ligne 14).

La présente description divulgue un réactif d'immunomonitoring comprenant au moins un peptide/dérivé peptidique tels que définis ci-dessus. En particulier,ledit réactif est inclus dans un coffret (kit). Ledit réactif d'immunomonitoring comprend en particulierun peptide, polypeptide ou une protéine de fusion tels que définis ci-dessus, éventuellement marqués ou complexés, notamment complexés à des molécules HLA marquées, par exemple biotinylées, sous la forme de complexes multimériques HLA/peptide comme des tétramères de complexes HLA/peptide, marqués.

La présente description divulgue, en outre, un procédé d'analyse de lymphocytes T CD4⁺ spécifiques de la cycline B1, caractérisé en ce qu'il comprend au moins les étapes suivantes :
- la mise en contact, *in vitro*, d'un échantillon cellulaire avec des complexes multimériques HLA /peptide marqués, notamment par un fluorochrome, lesdits complexes étant formés par la liaison de molécules HLA solubles avec au moins un peptide dérivé peptidique tels que définis ci-dessus, et
- l'analyse des cellules liées auxdits complexes HLA /peptide, notamment en cytométrie de flux.

En particulier, l'analyse des cellules (lymphocytes T CD4⁺) comprend le tri desdites cellules.

La présente description divulgue également un peptide comprenant un épitope T CD4⁺ de la cycline B1, capable de se lier à 3 à 5 ou au moins 6 molécules HLA II prépondérantes différentes tel que défini ci-dessus, à l'exception des peptides 212-226, 216-230 et 221-235, ainsi qu'un peptide modifié, polynucléotide, vecteur, cellule hôte et composition immunogène ou vaccinale dérivés.

Les polynucléotides, les vecteurs recombinants et les cellules transformées tels que définis ci-dessus, sont utiles notamment pour la production des peptides, polypeptides et protéines de fusion selon l'invention.

Les polynucléotides selon l'invention sont obtenus par les méthodes classiques, connues en elles-mêmes, en suivant les protocoles standards tels que ceux décrits dans Current Protocols in Molecular Biology (Frederick M. AUSUBEL, 2000, Wiley and son Inc, Library of Congress, USA*).* Par exemple, ils peuvent être obtenus par amplification d'une séquence nucléique par PCR ou RT-PCR, par criblage de banques d'ADN génomique par hybridation avec une sonde homologue, ou bien par synthèse chimique totale ou partielle. Les vecteurs recombinants sont construits et introduits dans des cellules hôtes par les méthodes classiques d'ADN recombinant et de génie génétique, qui sont connues en elles-mêmes.

Les peptides et leurs dérivés tels que définis ci-dessus, sont préparés par les techniques classiques connues de l'Homme du métier, notamment par synthèse en phase solide ou liquide ou par expression d'un ADN recombinant dans un système cellulaire approprié (eucaryote ou procaryote).

De manière plus précise :
- les peptides et leurs dérivés (variants, polypeptides multiépitopiques) peuvent être synthétisés en phase solide, selon la technique Fmoc, originellement décrite par Merrifield et al. (J. Am. Chem. Soc., 1964, 85: 2149-) et purifiés par chromatographie liquide haute performance en phase inverse,
- les lipopeptides peuvent être notamment préparés selon le procédé décrit dans les Demandes Internationales WO 99/40113 ou WO 99/51630.
- les peptides et dérivés tels que les variants, les polypeptides multiépitopiques et les protéines de fusion peuvent également être produits à partir des ADNc correspondants, obtenus par tout moyen connu de l'homme du métier ; l'ADNc est cloné dans un vecteur d'expression eucaryote ou procaryote et la protéine ou le fragment produits dans les cellules modifiées par le vecteur recombinant sont purifiés par tout moyen approprié, notamment par chromatographie d'affinité.

Outre les dispositions qui précèdent, l'invention comprend encore d'autres dispositions, qui ressortiront de la description qui va suivre, qui se réfère à des exemples de mise en œuvre de l'objet de la présente invention, avec référence aux dessins annexés dans lesquels :

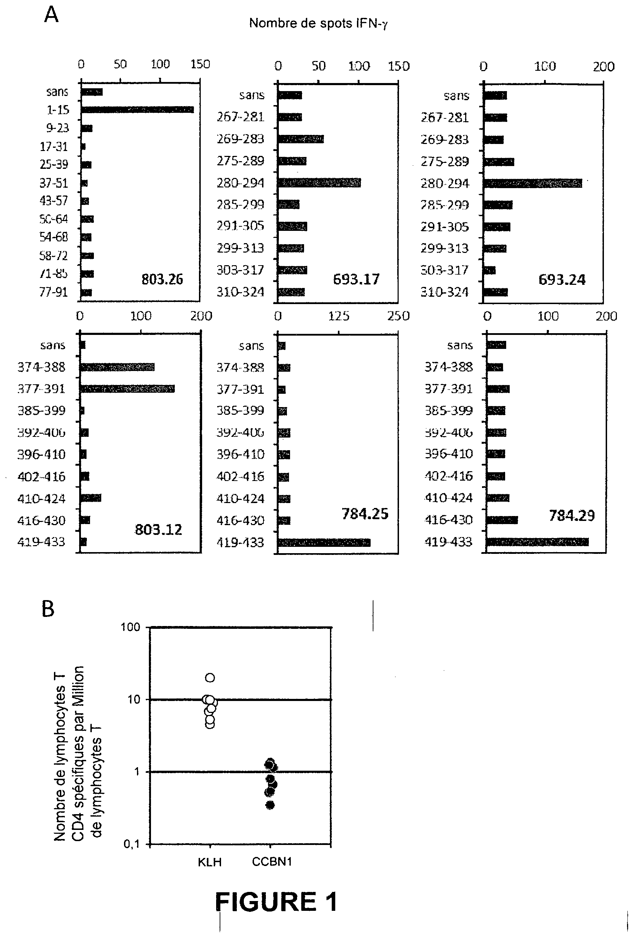

- la figure 1 illustre la spécificité et l'amplitude de la réponse des lymphocytes T CD4⁺ vis-à-vis de CCNB1. Les lignées de lymphocytes T CD4⁺ ont été obtenues par stimulation *in vitro* par des cellules dendritiques autologues préalablement chargées avec la protéine CCNB1. A. Exemples de lignées de lymphocytes T CD4⁺ spécifiques de peptides de CCNB1. B. Fréquences des lymphocytes T CD4+ spécifiques de la KLH et de CCNB1 dans le sang des donneurs.

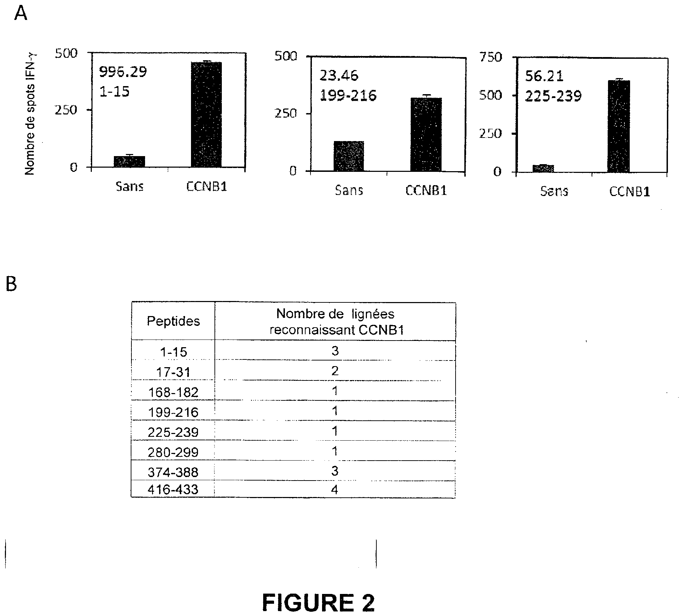

- la figure 2 illustre la reconnaissance de la protéine CCNB1 par les lignées de lymphocytes T CD4+ dirigés contre les peptides. Les lignées de lymphocytes T CD4+ ont été obtenues par stimulation *in vitro* par des cellules dendritiques autologues préalablement chargées avec des peptides de CCNB1. Les lignée de lymphocytes T CD4⁺ ont été mise en présence de cellules dendritiques autologues préalablement chargées avec CCNB1 et leur activation testée par Elispot IFN-gamma après 24h d'incubation. A. Exemples de lignées de lymphocytes T CD4⁺ spécifiques de peptides de CCNB1 reconnaissant CCNB1. Le nom de la lignée de lymphocytes T CD4⁺ et sa spécificité peptidique sont indiqués dans le cadran. B. Nombre de lignées de lymphocytes T CD4⁺ spécifiques des peptides reconnaissant CCNB1.

### Exemple 1 : Matériels et méthodes

### 1) Synthèse des peptides

Les peptides ont été synthétisés selon la stratégie Fmoc en synthèse parallèle sur phase solide au moyen d'une résine de type Rink amide, purifiés par HPLC et contrôlés par spectrométrie de masse (ES-MS). Tous les peptides ont leur N-terminal libre et leur C-terminal amidé.

### 2) Production et purification de la protéine CCNB1 humaine recombinante

Des bactéries E. Coli de la souche BL 21 ont été transformées par électroporation (2500V 4,8ms) avec le plasmide pET-28a-(+) (NOVAGEN) dans lequel avait été cloné le gène de la CCNB1 humaine. Après pré-culture en milieu LB 20 min à 37°C, les bactéries transformées ont été cultivées 48h à 37°C en milieu sélectif LB/Kanamycine puis l'expression de la Cycline B1 a été induite par incubation 21h à 15°C en présence d'IPTG. La protéine produite, qui possède une étiquette His, a été extraite par lyse cellulaire des bactéries (traitement aux lysozymes suivi d'un broyage mécanique puis d'un traitement avec des benzonases), puis purifiée par plusieurs chromatographies successives : une étape de chromatographie d'affinité sur colonne HisTrap (colonne de nickel, GE HEALTHCARE), une étape de chromatographie d'exclusion sur colonne Superdex 75 (GE HEALTHCARE), une étape de chromatographie d'échange d'ions sur colonne HiTrap SP HP (GE HEALTHCARE), et une étape d'élimination des endotoxines par chromatographie d'affinité sur colonne EndoTrap Blue (LONZA). La concentration de la protéine dans la solution finale a été déterminée par mesure de l'absorbance à 280 nm, et sa pureté vérifiée par spectrométrie de masse.

### 3) Préparation des cellules

Les prélèvements sanguins (Couche leuco plaquettaire) proviennent de l'Établissement Français du Sang (Centre de Rungis). Les cellules mononuclées du sang périphériques (PBMC) ont été isolés par gradient de ficoll. Les cellules dendritiques (DC) immatures ont été obtenues à partir des PBMC par différenciation des cellules adhérentes après 5 jours de culture en milieu AIM-V (LIFE TECHNOLOGIES) contenant 1000 U/ml d'IL-4 et 1000 U/ml de GM-CSF (R&D SYSTEMS). Les DC matures ont été obtenues à partir des DC immatures après avoir été cultivées pendant deux jours en présence de lipopolysaccharide (LPS). Les lymphocytes T CD4⁺ ont été purifiés à partir des PBMC à l'aide de microbilles magnétiques couplées à des anticorps anti-CD4 (MILTENYI BIOTEC). Le génotypage de HLA-DRB1 des donneurs a été réalisé par PCR séquence spécifique à l'aide du kit All Set⁺™ Gold SSP HLA-DRB1 (INVITROGEN).

### 4) Obtention de lignées de lymphocytes T CD4⁺ spécifiques de CCNB1ou des peptides

Les lymphocytes T CD4⁺ (1 à 3×I0⁵) ont été mis en culture en plaque 96 puits à fond rond avec des DC immatures autologues préalablement chargées avec 1,2 µM de CCNB1 et maturées en présence de LPS ou avec des DC matures autologues préalablement chargées avec des pools de peptides (10 µg/ml). La culture est faite dans du milieu IMDM (LIFE TECHNOLOGIES) supplémenté par du Sérum humain de groupe AB (milieu IMDM complet) contenant 1000 U/ml d'IL-6 (R&D SYSTEMS) et 10 ng/ml d'IL-12 (R&D SYSTEMS). La culture est stimulée au bout d'une semaine par ajout de 10000 à 30000 DC chargées avec la protéine CCNB1 ou les pools de peptides, de 20 U/ml d'IL-2 (R&D SYSTEMS) et de 10 ng/ml d'IL-7 (R&D Systems). Une autre stimulation est faite après 14 et éventuellement 21 jours de culture. Entre 5 et 7 jours après la dernière stimulation, un test de spécificité est réalisé par EliSpot. Chaque puits de culture constitue une lignée de lymphocytes T CD4⁺.

### 5) Evaluation de la spécificité de lymphocytes T CD4⁺ cultivés avec CCNB1 ou des peptides par EliSpot

L'anticorps anti-IFN-γ humain 1-D1K (MABTECH) est adsorbé à 2,5 µg/ml en PBS 1X sur des plaques 96 puits Multiscreen®-HA (MILLIPORE) par une incubation d'une nuit à 4°C. Les plaques sont ensuite saturées par une incubation de 2 heures à 37°C avec du milieu l'IMDM complet. Les lymphocytes T CD4⁺ sont incubés pendant 16 heures à 37°C dans ces plaques après avoir été lavés dans du milieu AIM-V/IL-7 (0,5 ng/ml d'IL-7). Les DC immatures autologues (5000 cellules/puits) préalablement chargées pendant 4 heures avec la protéine CCNB1 ou KLH (0.5 à 3µ M) ou des PBMC (50 000 cellules/puits) incubés en présence de 10 µg/ml de peptides sont utilisées comme cellules présentatrices. Après l'incubation, les plaques sont lavées avec de l'eau distillée, du PBS /Tween 0,05 % et du PBS. 100 µl/puits d'Ac anti-IFN-γ humain 7-B6-1 biotinylé (MABTECH) à 0,25 µg/ml en PBS 1X/BSA 1 % est ajouté dans les plaques qui sont incubées 1h 30min à 37°C. Après plusieurs lavages en PBS ou PBS/tween 0,05 %, la sécrétion d'IFN-γ est mise en évidence par l'ajout de l'Extravidine-Phosphatase Alcaline (SIGMA) et de NBT/BCIP. Après 5 à 10 minutes d'incubation, la réaction est stoppée par lavage à l'eau courante. Après séchage, les spots sont comptés sur un lecteur Elispot (AID). Une lignée de lymphocytes T CD4⁺ est considérée comme spécifique de l'antigène si le nombre de spots dans les puits contenant l'antigène est au moins deux fois plus élevé que le puits ne contenant pas l'antigène, la différence entre les 2 types de puits étant au moins supérieure à 25. La fréquence des lymphocytes T CD4⁺ présents dans le sang des donneurs est estimée en utilisant la distribution de la loi de Poisson selon la formule : Fréquence = -Ln ((Nombre de lignées non spécifiques/Nombre total de lignées testées))/ (Nombre de Lymphocytes CD4⁺ incubés dans chaque puits).

### 6) Purification des molécules HLA de classe II

Des cellules lymphoblastoïdes transformées par le virus EBV homozygotes pour les molécules HLA de classe II sont utilisées comme source des molécules HLA de classe II (Texier et al., J. Immunol., 2000, 164, 3177-3184 ; Texier et al., Eur. J. Immunol., 2001, 31, 1837-1846 ; Castelli et al., J. Immunol., 2002, 169, 6928-6934). Les molécules HLA-DR et HLA-DP sont purifiées par chromatographie d'affinité au moyen des anticorps monoclonaux anti-HLA-DR L243 (ATCC, Rockville, USA) et anti-HLA-DP B7/21 couplés sur un gel de proteine A sepharose CL 4B gel (PHARMACIA).

### 7) Mesure de l'affinité relative des peptides pour les molécules HLA de classe II

L'affinité des peptides est évaluée par compétition entre un peptide biotinylé et le peptide à tester vis-à-vis des molécules HLA de classe II. La révélation de complexes est faite par ELISA. Ces tests sont décrits décrits dans le brevet américain US 6,649,166 et la Demande Internationale PCT WO 03/040299, respectivement pour les molécules HLA-DR et HLA-DP4. La mise en œuvre de ces tests pour mesurer l'activité de liaison de peptides issus de différents antigènes, est illustrée dans le brevet américain US 6,649,166 et les Demandes Internationale PCT WO 02/090382, WO 03/040299 et WO 2004/014936.

De manière plus précise, les molécules HLA de classe II sont diluées dans un tampon Phosphate 10 mM, 150 mM NaCl, 1 mM Dodecylmaltoside (DM) et 10 mM citrate avec une concentration fixe du peptide biotinylé et des dilutions du peptide à tester. Les plaques sont incubées 24 h à 72 h. Après neutralisation par 50 µL de tampon 450 mM Tris HCl pH=7,5, 0,003% thimerosal, 0,3% BSA, 1 mM DM, les échantillons sont transférés sur des plaques ELISA 96 puits (Maxisorp®, NUNC), sur lesquelles les anticorps L243 (anti-HLA-DR) ou B7/21 (anti-HLA-DP) ont été adsorbés à une concentration de 10 µg/ml. Ces plaques ont été également saturées par un tampon 100 mM Tris HCl pH=7,5, 0,3 % BSA, 0,003 % thimerosal. Après 2 heures d'incubation, les plaques ELISA sont lavées. La présence dans les puits du peptide biotinylé est révélée par l'incubation successive d'un conjugué streptavidine-phosphatase alkaline (GE HEALTHCARE) et de 4-methylumbelliferyl phosphate comme substrat (SIGMA). La fluorescence émise est mesurée à 450 nm après excitation à 365 nm au moyen d'un spectrofluorimètre Gemini (MOLECULAR DEVICES). La liaison maximale est donnée par les puits ne contenant pas de compétiteur et le bruit de fond est mesuré dans les puits ne comportant pas de molécule HLA. Pour chaque peptide, la concentration inhibant 50 % de la liaison maximale (IC50) est évaluée. Chaque expérience est contrôlée par un peptide de référence qui est la forme non biotinylée du peptide traceur. Leur séquence et leur valeur d'IC50 sont les suivantes : HA 306-318 (PKYVKQNTLKLAT ; SEQ ID NO : 2) pour DRB1*01:01 (1 nM), DRB1*04:01 (6 nM), DRB1*11:01 (5 nM) et DRB5*01:01 (3 nM), YKL (AAYAAAKAAALAA; SEQ ID NO: 3) pour DRB1*07:01 (10 nM), A3 152-166 (EAEQLRAYLDGTGVE; SEQ ID NO : 4) pour DRB1*15:01 (30 nM), MT 2-16 (AKTIAYDEEARRGLE; SEQ ID NO: 5) pour DRB1*03:01 (121 nM), B1 21-36 (TERVRLVTRHIYNREE ; ; SEQ ID NO : 6) pour DRB1*13:01 (51 nM), LOL 191-210 (ESWGAVWRIDTPDKLTGPFT; SEQ ID NO : 7) pour DRB3*01:01 (28 nM), E2/E168 (AGDLLAIETDKATI; SEQ ID NO : 8) pour DRB4*01:01 (10 nM) et Oxy 271-287 (EKKYFAATQFEPLAARL; SEQ ID NO : 9) pour DPB1*04:01 (7 nM) et DPB1*04:02 (6 nM). Les données sont exprimées sous la forme d'affinité relative: IC50 du peptide/ IC50 du peptide de référence. Une valeur d'affinité relative inférieure à 100 correspond à un peptide ligand de la molécule HLA de classe II. Chaque valeur est la moyenne d'au moins deux expériences indépendantes.

### Exemple 2 : Choix de la séquence des peptides de CNBB1

Les molécules HLA de classe II et en particulier les molécules HLA-DR et HLA-DP fixent les peptides antigéniques par plusieurs résidus d'ancrage présents sur la séquence des peptides. Ces peptides possèdent un résidu hydrophobe ou aromatique dans leur partie N terminale qui constitue le résidu d'ancrage principal, logé dans la poche P1 de la molécule HLA de classe II. De manière à optimiser la liaison des peptides aux molécules HLA de classe II, les chevauchements des peptides qui couvrent l'intégralité de la séquence de CCNB1 ont été choisis de telle façon qu'ils possèdent tous un résidu hydrophobe ou aromatique dans leur partie N-terminale. Ces peptides sont la séquence est présentée dans le Tableau III ont été synthétisés.

**Tableau III : Peptides chevauchants issus de la protéine CCNB1 (SEQ ID NO : 10 à 84)**

| **Numérotation*** | **Séquence** | **Numérotation** | **Séquence** |
|---|---|---|---|
| 1-15 | MA**L**RVTRNSKINAEN | 241-255 | KK**M**LQLVGVTAMFIA |
| 9-23 | SK**I**NAENKAKINMAG | 244-258 | LQ**L**VGVTAMFIASKY |
| 17-31 | AK**I**NMAGAKRVPTAP | 250-264 | TA**M**FIASKYEEMYPP |
| 25-39 | KR**V**PTAPAATSKPGL | 256-270 | SK**Y**EEMYPPEIGDFA |
| 37-51 | PG**L**RPRTALGDIGNK | 260-274 | EM**Y**PPEIGDFAFVTD |
| 43-57 | TA**L**GDIGNKVSEQLQ | 264-278 | PE**I**GDFAFVTDNTYT |
| 50-64 | NK**V**SEQLQAKMPMKK | 267-281 | GD**F**AFVTDNTYTKHQ |
| 54-68 | EQ**L**QAKMPMKKEAKP | 269-283 | FA**F**VTDNTYTKHQIR |
| 58-72 | AK**M**PMKKEAKPSATG | 275-289 | NT**Y**TKHQIRQMEMKI |
| 71-85 | TGK**V**IDKKLPKPLEK | 280-294 | HQ**I**RQMEMKILRALN |
| 77-91 | KK**L**PKPLEKVPMLVP | 285-299 | ME**M**KILRALNFGLGR |
| 81-95 | KP**L**EKVPMLVPVPVS | 291-305 | RA**L**NFGLGRP LPLHF |
| 87-101 | PM**L**VPVPVSEPVPEP | 299-313 | RP**L**PLHFLRRASKIG |
| 96-110 | EP**V**PEPEPEPEPEPV | 303-317 | LH**F**LRRASKIGEVDV |
| 108-122 | EP**V**KEEKLSPEPILV | 310-324 | SK**I**GEVDVEQHTLAK |
| 113-127 | EK**L**SPEPILVDTASP | 315-329 | VD**V**EQHTLAKYLMEL |
| 118-132 | EP**I**LVDTASPSPMET | 320-334 | HT**L**AKYLMELTMLDY |
| 128-142 | SP**M**ETSGCAPAEEDL | 323-337 | AK**Y**LMELTMLDYDMV |
| 140-154 | ED**L**CQAFSDVILAVN | 327-341 | ME**L**TMLDYDMVHFPP |
| 144-158 | QA**F**SDVILAVNDVDA | 332-346 | LD**Y**DMVHFPPSQIAA |
| 147-161 | SD**V**ILAVNDVDAEDG | 338-352 | H**F**PPSQIAAGAFCLA |
| 151-165 | LA**V**NDVDAEDGADPN | 342-356 | SQ**I**AAGAFCLALKIL |
| 164-178 | PN**L**CSEYVKDIYAYL | 347-361 | GA**F**CLALKILDNGEW |
| 168-182 | SE**Y**VKDIYAYLRQLE | 351-365 | LA**L**KILDNGEWTPTL |
| 172-186 | KD**I**YAYLRQLEEEQA | 359-373 | GE**W**TPTLQHYLSYTE |
| 179-193 | RQ**L**EEEQAVRPKYLL | 363-377 | PT**L**QHYLSYTEESLL |
| 185-199 | QA**V**RPKYLLGREVTG | 366-380 | QH**Y**LSYTEESLLPVM |
| 189-203 | PK**Y**LLGREVTGNMRA | 369-383 | LS**Y**TEESLLPVMQHL |
| 195-209 | RE**V**TGNMRAILIDWL | 374-388 | ES**L**LPVMQHLAKNW |
| 199-213 | GN**M**RAILIDWLVQVQ | 377-391 | LP**V**MQHLAKNWMVN |
| 202-216 | RA**I**LIDWLVQVQMKF | 385-399 | KN**V**VMVNQGLTKHMT |
| 207-221 | DW**L**VQVQMKFRLLQE | 392-406 | QG**L**TKHMTVKNKYAT |
| 212-226 | VQ**M**KFRLLQETMYMT | 396-410 | KH**M**TVKNKYATSKHA |
| 216-230 | FR**L**LQETMYMTVSII | 402-416 | NK**Y**ATSKHAKISTLP |
| 221-235 | ET**M**YMTVSIIDRFMQ | 410-424 | AK**I**STLPQLNSALVQ |
| 225-239 | MT**V**SIIDRFMQNNCV | 416-430 | PQ**L**NSALVQDLAKAV |
| 231-245 | DR**F**MQNNCVPKKMLQ | 419-433 | NSA**L**VQDLAKAVAKV |
| 237-251 | NC**V**PKKMLQLVGVTA | | |

| | | | |
|---|---|---|---|
| * la numérotation est réalisée en référence à la séquence CNBB1 humaine (SEQ ID NO : 1) **Le résidu hydrophobe ou aromatique est en gras | | | |

### Exemple 3 : Obtention de lignées de lymphocytes T CD4⁺ spécifiques de la protéine CCNB1

8 donneurs sains comportant des molécules HLA variées et la plupart très fréquentes dans la population caucasienne ont été sélectionnés. Des lignées de lymphocytes T CD4⁺ ont été obtenues par co-culture des lymphocytes T CD4⁺ avec des cellules dendritiques autologues préalablement chargées avec la protéine CCNB1, produite par voie recombinante dans *E. Coli.* A l'issue de la culture, chaque lignée a été testée par Elispot pour sa capacité à reconnaître des pools de peptides qui couvrent la séquence de la protéine CCNB1 puis dans un deuxième test, les peptides individuels contenus dans le pool reconnu par les lignées. Afin de s'assurer de la capacité de chaque donneur à pouvoir répondre, des lignées de lymphocytes T CD4⁺ spécifiques de la KLH ont été également produites.

Le Tableau IV indique les peptides reconnus par les lymphocytes T CD4⁺ spécifiques de la cycline B1 et le nombre de lignées spécifiques qui leur sont associés.

**Tableau IV : Spécificité peptidique des lignées de lymphocytes T spécifiques de CCNB1**

| | Typage | peptides | | | | | | | | | | | | | | | |
|---|---|---|---|---|---|---|---|---|---|---|---|---|---|---|---|---|---|
| Donneurs | HLA-DRB1 | 1-15 | 17-31 | 25-39 | 118-132 | 280-294 | 285-299 | 303-317 | 315-329 | 320-334 | 323-337 | 374-388 | 377-391 | 410-424 | 416-430 | 419-433 | TOTAL |
| 693 | 01:02/13:01 | | | | | | 3 | 1 | | | | | | | | | 4 |
| 694 | 04:01/15:01 | | | | 1 | | 1 | | | | 1 | | | | 1 | | 4 |
| 703 | 07:01 /13:02 | | | | | 1 | 2 | | | | | | | | | | 3 |
| 721 | 03:01/11:01 | | | | | | | | | | | | | 2 | | | 2 |
| 593 | 01:01/08:01 | | | | | | 1 | | 1 | 1 | | | | | | | 3 |
| 784 | 07:01/11:01 | 2 | 1 | 1 | | | | | | | | | | | | 5 | 9 |
| 795 | 11:01/15:01 | | | | | | | | | | | | | 1 | | | 1 |
| 803 | 04:04/04:01 | 2 | | | | | | | | | | 1 | 1 | | | | 4 |
| | Total | 4 | 1 | 1 | 1 | 1 | 7 | 1 | 1 | 1 | 1 | 1 | 1 | 3 | 1 | 5 | |

15 peptides différents correspondant à des épitopes T CD4⁺ immunodominants *in vitro* de CCNB1 ont été identifiés (Tableau IV). 3 peptides sont communs à au moins deux individus (Tableau IV). Ces 15 épitopes T immunodominants *in vitro* sont différents des épitopes T connus (épitopes 215-229, 219-233 et 223-234 ; Vella et al., Proc. Natl. Acad. Sci. U S A, 2009, 106, 14010-14015; Demande US 2011/0280897) et situés en dehors de la région correspondante de CNBB1 (région 215-233 ; Vella et al., Proc. Natl. Acad. Sci. U S A, 2009, 106, 14010-14015; Demande US 2011/0280897). En outre, les peptides identifiés participent à la réponse contre CCNBlétant donné que les lymphocytes T CD4+ ont été générés par stimulation avec la protéine CCNB1. Des exemples de lignée de lymphocytes T CD4⁺ spécifiques de CCNB1 et de peptides de cette protéine sont présentés (Figure 1A).

Sur la base du nombre de lignées obtenues, la fréquence des lymphocytes T CD4⁺ préexistants dans le sang des donneurs a été évaluée. Pour ces 8 donneurs, cette fréquence moyenne est de 0,82 pour CCNBlet d'au moins de 9,2 pour la KLH et, tous les donneurs étant répondeurs aux 2 protéines (Fig 1B). Cette valeur de fréquence de précurseurs correspond à des protéines immunogènes (Delluc et al., Blood, 2010, 116, 4542-4545; Delluc et al., Faseb J., 2011, 25, 2040-2048).

### Exemple 4 : Affinité des peptides de CCNB1 pour les molécules HLA de classe II

L'ensemble des peptides chevauchants couvrant la séquence de la cycline B1 ont été testés pour leur capacité à se lier aux molécules HLA de classe II par un test de liaison en compétition comme décrit à l'exemple 1. Les données sont présentées dans 3 tableaux différents en fonction des peptides : les peptides ayant été trouvés comme épitopes T immunodominants *in vitro* de CCNB1 lors des expériences précédentes (Tableau V, les peptides supplémentaires ayant une large spécificité de liaison pour les molécules HLA de classe II (Tableau VI), et le reste des peptides (Tableau VII). Pour chaque peptide, la concentration inhibant 50% de la liaison maximale (IC50) est évaluée. Les données sont exprimées sous la forme d'affinité relative: IC50 du peptide/ IC50 du peptide de référence. Une valeur d'affinité relative inférieure à 100 correspond à un peptide ligand de la molécule HLA de classe II. Une valeur d'affinité relative inférieure à 20 correspond à un peptide ayant une forte affinité (valeur en gras). Chaque valeur est la moyenne d'au moins deux expériences indépendantes. Les peptides en gras ont été sélectionnés pour les expériences suivantes.

**Tableau V : Activité de liaison aux molécules HLA de classe II des épitopes T immunodominants in vitro**

| **Peptides** | **DR1** | **DR3** | **DR4** | **DR7** | **DR11** | **DR13** | **DR15** | **DRB3** | **DRB4** | **DRB5** | **DP401** | **DP402** | Nb HLA II |
|---|---|---|---|---|---|---|---|---|---|---|---|---|---|
| **1-15** | 100 | >825 | 46 | **2** | 22 | 58 | **5** | >362 | >961 | 374 | >1 357 | >1 733 | **6** |
| **17-31** | **5** | 833 | >1 771 | **3** | **2** | 21 | 120 | >362 | >961 | **11** | >1 357 | >1 733 | **5** |
| **280-294** | 1 225 | **0,2** | **0,4** | 462 | 2 000 | >1 949 | **0,3** | 53 | >961 | **4** | >1 357 | >1 733 | **5** |
| **285-299** | **1** | 51 | **7** | **0,1** | **4** | **1** | **0,03** | >362 | **18** | **2** | 30 | **4** | **11** |
| **303-317** | 495 | **3** | 1 394 | 849 | **1** | **1** | **17** | >362 | >961 | 1 333 | >1 357 | >1 733 | **4** |
| **320-334** | **16** | **1** | **1** | **18** | **2** | **10** | **3** | 24 | **18** | **17** | **8** | **3** | **12** |
| **323-337** | **4** | **2** | **0,2** | **8** | **0,4** | >1 949 | **2** | 41 | **7** | **2** | **1** | **6** | **11** |
| **374-388** | 837 | 764 | 91 | **3** | **0,3** | **6** | **19** | >362 | 424 | 91 | >1 357 | >1 733 | **6** |
| **410-424** | 89 | >825 | 408 | >1 036 | **1** | 94 | **12** | >362 | 966 | >3 467 | >1 357 | 1118 | **4** |
| **416-430** | 693 | **9** | 54 | 47 | 123 | >1 949 | 71 | 3 | 1 800 | 548 | 59 | 74 | **7** |
| **419-433** | 155 | **1** | 333 | 183 | **3** | 37 | **7** | **7** | >961 | 112 | >1 357 | 148 | **5** |
| **25-39** | >11 262 | >825 | >1 771 | >1 036 | >1 949 | >1 949 | >329 | >362 | >961 | >3 467 | >1 357 | >1 733 | 0 |
| **118-132** | 6359 | **0,4** | **0,3** | >1 036 | >1 949 | >1 949 | 400 | **12** | >961 | >3 467 | >1 357 | >1 733 | 3 |
| **315-329** | 2449 | >825 | 149 | **6** | 632 | 211 | **7** | >362 | >961 | 224 | >1 357 | >1 733 | 2 |
| **377-391** | 894 | 177 | 373 | **13** | **3** | **2** | >329 | >362 | >961 | 4 082 | >1 357 | >1 733 | 3 |

Le Tableau V montre que sur les 15 peptides identifiés comme immunodominants *in vitro,* 11 peptides se lient à au moins 4 molécules HLA de classe II différentes, 3 peptides se liant à plus de 11 molécules sur les 12 testées.

Le Tableau VI présente les résultats d'autres peptides qui possèdent également une large spécificité de liaison aux molécules HLA de classe II. Ces peptides se lient à au moins 6 molécules différentes.

**Tableau VI : Activité de liaison aux molécules HLA de classe II d'autres peptides que les peptides immunodominants in vitro ayant une large spécificité de liaison**

| **peptides** | **DR1** | **DR3** | **DR4** | **DR7** | **DR11** | **DR13** | **DR15** | **DRB3** | **DRB4** | **DRB5** | **DP401** | **DP402** | Nb HLA II |
|---|---|---|---|---|---|---|---|---|---|---|---|---|---|
| **168-182** | 22 | 97 | 163 | 346 | **3** | **12** | **12** | 30 | >961 | **3** | **159** | 127 | 7 |
| **199-213** | 27 | **1** | 56 | **10** | 77 | >1 949 | **1** | **9** | **9** | 58 | 36 | 24 | 11 |
| **202-216** | 1 225 | **1** | 59 | **9** | 400 | >1 949 | **4** | **2** | **20** | 55 | 47 | **14** | 9 |
| **207-221** | 38 | **0,5** | >1 771 | **7** | **0,4** | **1** | **7** | 160 | 49 | **5** | >1 357 | 171 | 8 |
| **212-226** | **1** | **1** | **2** | 40 | **5** | **1** | 46 | 34 | 683 | **2** | **110** | **9** | 10 |
| **216-230** | **7** | **1** | **2** | **0,5,** | **14** | >1 949 | **3** | **11** | 22 | 50 | **2** | **1** | 11 |
| **221-235** | 89 | **0,5** | 37 | **1** | **1** | 59 | **0,3** | 22 | **7** | **1** | 59 | 68 | 12 |
| **225-239** | 24 | **1** | 100 | **5** | 31 | >1 949 | **2** | **9** | **6** | 79 | 72 | 35 | 11 |
| **241-255** | **3** | **15** | **1** | **0,1** | 90 | >1 949 | **1** | >362 | 141 | **1** | 424 | 35 | 8 |
| **244-258** | **2** | **9** | 141 | **1** | 23 | >1 949 | **1** | 267 | 98 | **1** | 77 | 32 | 9 |
| **250-264** | 22 | **4** | 70 | **4** | **6** | **3** | **9** | 34 | >961 | 82 | **0,1** | **1** | 11 |
| **267-281** | 1 500 | **0,2** | **1** | 22 | 1333 | 62 | 86 | 38 | >961 | 24 | >1 357 | >1 733 | 7 |
| **299-313** | 63 | 168 | 624 | 35 | **0,1** | **0,2** | 200 | >362 | **13** | 26 | >1 357 | >1 733 | 6 |
| **342-356** | 46 | >825 | 163 | **20** | **3** | >1949 | 21 | 239 | 57 | 163 | 79 | 25 | 7 |
| **363-377** | 28 | **1** | 76 | **1** | 45 | >1 949 | **1** | **11** | **5** | 24 | 22 | 50 | 11 |
| **366-380** | 32 | 119 | 65 | **1** | 55 | >1 949 | 47 | 32 | >961 | 471 | **0,2** | **0,3** | 8 |

Le Tableau VII présente les résultats des peptides restants.

**Tableau VII : Activité de liaison aux molécules HLA de classe II du reste des peptides couvrant la cycline B1**

| **peptides** | **DR1** | **DR3** | DR4 | **DR7** | **DR11** | **DR13** | **DR15** | **DRB3** | **DRB4** | **DRB5** | **DP401** | **DP402** | Nb HLA II |
|---|---|---|---|---|---|---|---|---|---|---|---|---|---|
| 9-23 | >11 262 | 22 | >1 771 | >1 036 | 490 | 146 | >329 | >362 | >961 | 1 000 | >1 357 | >1 733 | 1 |
| 37-51 | >11 262 | >825 | >1 771 | >1 036 | >1 949 | 422 | >329 | >362 | >961 | >3 467 | >1 357 | >1 733 | 0 |
| 43-57 | >11 262 | >825 | >1 771 | >1 036 | >1 949 | 1 789 | 300 | >362 | >961 | >3 467 | >1 357 | >1 733 | 0 |
| 50-64 | >11 262 | >825 | >1 771 | >1 036 | 1 549 | 258 | >329 | >362 | >961 | 51 | >1 357 | >1 733 | 1 |
| 54-68 | 3 742 | >825 | >1 771 | 808 | 1 414 | 105 | >329 | >362 | >961 | 35 | >1 357 | >1 733 | 1 |
| 58-72 | >11 262 | >825 | >1 771 | >1 036 | 40 | 683 | >329 | >362 | >961 | >3 467 | >1 357 | >1 733 | 1 |
| 71-85 | >11 262 | 228 | >1 771 | >1 036 | >1 949 | >1 949 | >329 | >362 | >961 | 250 | >1 357 | >1 733 | 0 |
| 77-91 | >11 262 | >825 | >1 771 | >1 036 | 1 633 | >1 949 | >329 | >362 | 75 | >3 467 | 212 | 474 | 1 |
| 81-95 | 524 | >825 | 500 | >1 036 | >1 949 | >1 949 | >329 | >362 | 141 | >3 467 | >1 357 | >1 733 | 0 |
| 87-101 | 1 500 | >825 | 96 | 231 | >1 949 | >1 949 | 134 | >362 | 748 | >3 467 | >1 357 | >1 733 | 1 |
| 96-110 | >11 262 | >825 | >1 771 | >1 036 | >1 949 | >1 949 | >329 | >362 | >961 | >3 467 | >1 357 | >1 733 | 0 |
| 108-122 | 894 | >825 | >1 771 | >1 036 | >1 949 | >1 949 | >329 | >362 | >961 | >3 467 | >1 357 | >1 733 | 0 |
| 113-127 | 6 000 | **6** | 55 | >1 036 | >1 949 | >1 949 | >329 | 151 | 231 | >3 467 | 72 | 71 | 4 |
| 128-142 | 3586 | >825 | >1 771 | >1 036 | >1 949 | >1 949 | >329 | >362 | >961 | 467 | >1 357 | >1 733 | 0 |
| 140-154 | >11 262 | 161 | 1107 | >1 036 | >1 949 | >1 949 | 207 | >362 | >961 | >3 467 | >1 357 | >1 733 | 0 |
| 144-158 | 1 732 | **8** | **12** | >1 036 | >1 949 | >1 949 | 46 | **18** | >961 | >3 467 | 51 | 129 | 5 |
| 147-161 | >11 262 | >825 | 54 | >1 036 | >1 949 | >1 949 | **7** | >362 | >961 | 467 | >1 357 | >1 733 | 2 |
| 151-165 | >11 262 | >825 | 667 | >1 036 | >1 949 | >1 949 | >329 | >362 | >961 | >3 467 | >1 357 | >1 733 | 0 |
| 164-178 | 2 828 | >825 | 527 | 73 | >1 949 | >1 949 | 28 | 53 | 40 | 500 | 274 | 625 | 4 |
| 172-186 | 42 | 33 | 187 | 1 600 | **1** | **5** | **11** | >362 | >961 | 775 | >1 357 | >1 733 | 5 |
| 179-193 | 9000 | >825 | >1 771 | 271 | >1 949 | 943 | >329 | >362 | >961 | 2160 | >1 357 | >1 733 | 0 |
| 185-199 | 59 | >825 | 260 | >1 036 | 22 | **4** | >329 | >362 | >961 | 1 667 | >1 357 | >1 733 | 3 |
| 189-203 | **17** | 764 | 34 | >1 036 | **3** | >1 949 | >329 | >362 | >961 | 129 | >1 357 | >1 733 | 3 |
| 195-209 | 55 | **1** | 260 | 173 | 1 183 | 45 | 30 | **20** | 23 | 1414 | 725 | 791 | 5 |
| 231-245 | 11 | >825 | 52 | 036 | 2 000 | >1 949 | >329 | >362 | >961 | 1 581 | >1 357 | >1 733 | 2 |
| 237-251 | **4** | >825 | 179 | 115 | 87 | >1 949 | 65 | >362 | 1 400 | 112 | >1 357 | >1 733 | 3 |
| 256-270 | **3** | >825 | 456 | 61 | 1 366 | >1 949 | 153 | >362 | >961 | 603 | 894 | >1 733 | 2 |
| 264-278 | 7 000 | 85 | **2** | 86 | 1 667 | >1 949 | 300 | **19** | >961 | 61 | >1 357 | >1 733 | 5 |
| 269-283 | 1342 | **0,1** | **0,4** | 577 | 2 191 | >1 949 | **17** | 34 | >961 | 42 | >1 357 | >1 733 | 5 |
| 275-289 | 173 | >825 | 456 | 43 | **4** | 158 | 85 | >362 | 80 | 50 | >1 357 | >1 733 | 5 |
| 291-305 | 1 | >825 | 1 414 | **3** | 1000 | >1 949 | 65 | >362 | >961 | **4** | >1 357 | >1 733 | 4 |
| 310-324 | >11 262 | 306 | >1 771 | 036 | 190 | 1 800 | >329 | >362 | >961 | 1 500 | >1 357 | >1 733 | 0 |
| 327-341 | 418 | 32 | 22 | 126 | 216 | >1 949 | **10** | >362 | 139 | 163 | **2** | 47 | 5 |
| 332-346 | 224 | >825 | 289 | **20** | 511 | >1 949 | 36 | >362 | >961 | 1 581 | >1 357 | >1 733 | 2 |
| 338-352 | 598 | >825 | >1 771 | 1 400 | >1 949 | >1 949 | 257 | >362 | >961 | >3 467 | >1 357 | 188 | 0 |
| 347-361 | >11 262 | >825 | >1 771 | >1 036 | **9** | >1 949 | **1** | >362 | >961 | >3 467 | >1 357 | >1 733 | 2 |
| 351-365 | 648 | >825 | 1 000 | 624 | 2 333 | >1 949 | 52 | >362 | >961 | 3 055 | >1 357 | 510 | 2 |
| 359-373 | 548 | >825 | 500 | **0,4** | 22 | >1 949 | **0,2** | >362 | >961 | 224 | >1 357 | >1 733 | 3 |
| 369-383 | 212 | >825 | 58 | 91 | 45 | >1 949 | 350 | 72 | >961 | 1 379 | **4** | **1** | 6 |
| | | | | | | | | | | | | | |
| 385-399 | 4 500 | 228 | 986 | >1 036 | 766 | 37 | **0,02** | >362 | >961 | 52 | 116 | **2** | **4** |
| 392-406 | 262 | 408 | 577 | 533 | 693 | **7** | 129 | >362 | >961 | 47 | >1 357 | >1 733 | 2 |
| 396-410 | 7483 | >825 | 408 | 533 | **7** | **9** | 229 | >362 | >961 | 187 | >1 357 | >1 733 | 2 |
| 402-416 | 367 | >825 | 707 | **9** | **4** | >1 949 | >329 | >362 | >961 | 1 732 | >1 357 | >1 733 | 2 |

Certains des peptides du Tableau VII sont capables de se fixer à 4 ou 5 molécules HLA de classe II.

Les peptides du Tableau VI et les 11peptides immunodominants *in vitro* qui se lient à au moins 4 molécules HLA de classe II différentes (Tableau V) ont été retenus pour des études supplémentaires. En revanche, aucun des peptides du Tableau VII n'a été retenu, le nombre de peptides pouvant être évalué étant limité.

### Exemple 5 : Induction de lignées de lymphocytes T CD4⁺ spécifiques de peptides de la cycline B1

Les peptides retenus pour les tests d'induction de lignées de lymphocytes T **CD4⁺** sont décrits au Tableau VIII. Pour 7 peptides, les séquences chevauchantes ont été combinées entre elles, formant des peptides plus longs allant jusqu'à 20 acides aminés.

**Tableau VIII : Séquences des peptides testés pour leur capacité d'induction de lymphocytes T CD4+ spécifiques**

| **séquences** | **Combinaison** | Séquence | Taille |
|---|---|---|---|
| 1-15 | | MALRVTRNSKINAEN | 15 |
| 17-31 | | AKINMAGAKRVPTAP | 15 |
| 168-182 | | SEYVKDIYAYLRQLE | 15 |
| 199-216 | 199-213, 202-216 | GNMRAILIDWLVQVQMKF | 18 |
| 207-221 | | DWLVQVQMKFRLLQE | 15 |
| 212-226 | | VQMKFRLLQETMYMT | 15 |
| 216-230 | | FRLLQETMYMTVSII | 15 |
| 221-235 | | ETMYMTVSIIDRFMQ | 15 |
| 225-239 | 241-255,244-258 | MTVSIIDRFMQNNCV | 15 |
| 241-258 | | KKMLQLVGVTAMFIASKY | 18 |
| 250-264 | | TAMFIASKYEEMYPP | 15 |
| 267-281 | 280-294, 285-299 299-313, 303-317 320-334, 323-337 | GDFAFVTDNTYTKHQ | 15 |
| 280-299 | | HQIRQMEMKILRALNFGLGR | 20 |
| 299-317 | | RPLPLHFLRRASKIGEVDV | 19 |
| 320-337 | | HTLAKYLMELTMLDYDMV | 18 |
| 342-356 | 363-377, 366-380 | SQIAAGAFCLALKIL | 15 |
| 363-380 | | PTLOHYLSYTEESLLPVM | 18 |
| 374-388 | | ESLLPVMQHLAKNVV | 15 |
| 410-424 | 416-430, 419-433 | AKISTLPQLNSALVQ | 15 |
| 416-433 | | PQLNSALVQDLAKAVAKV | 18 |

13 donneurs sains comportant des molécules HLA variées et la plupart très fréquentes dans la population caucasienne ont été sélectionnés (Tableau IX).

Des lignées de lymphocytes T CD4⁺ ont été obtenues par co-culture des lymphocytes T CD4⁺ avec des cellules dendritiques autologues préalablement chargées avec des pools de peptides. Après amplification des lymphocytes T, chaque lignée a été testée par Elispot (Tableau IX). Les résultats sont reportés sous la forme du nombre de fois où une réponse à un peptide est observée. Tous les peptides induisent une réponse en lymphocytes T mais l'intensité de la réponse et le nombre de répondeurs sont très variables. 9 peptides activent des lymphocytes T chez plus de la moitié des donneurs.

Toutefois, de façon remarquable, les peptides les plus performants, capables d'induire la réponse T CD4⁺ spécifique la plus efficace (intensité de réponse supérieure à 2,5% et pouvant atteindre 6,1% et fréquence de répondeurs supérieure à 65 % et pouvant atteindre 85 %) dans une population d'individus portant des molécules HLA II variées, comprenant toutes les molécules HLA-DRB1 les plus fréquentes dans la population caucasienne, couvrant à elles seules 80 % des individus de la population caucasienne correspondent à des peptides immunodominants *in vitro.*

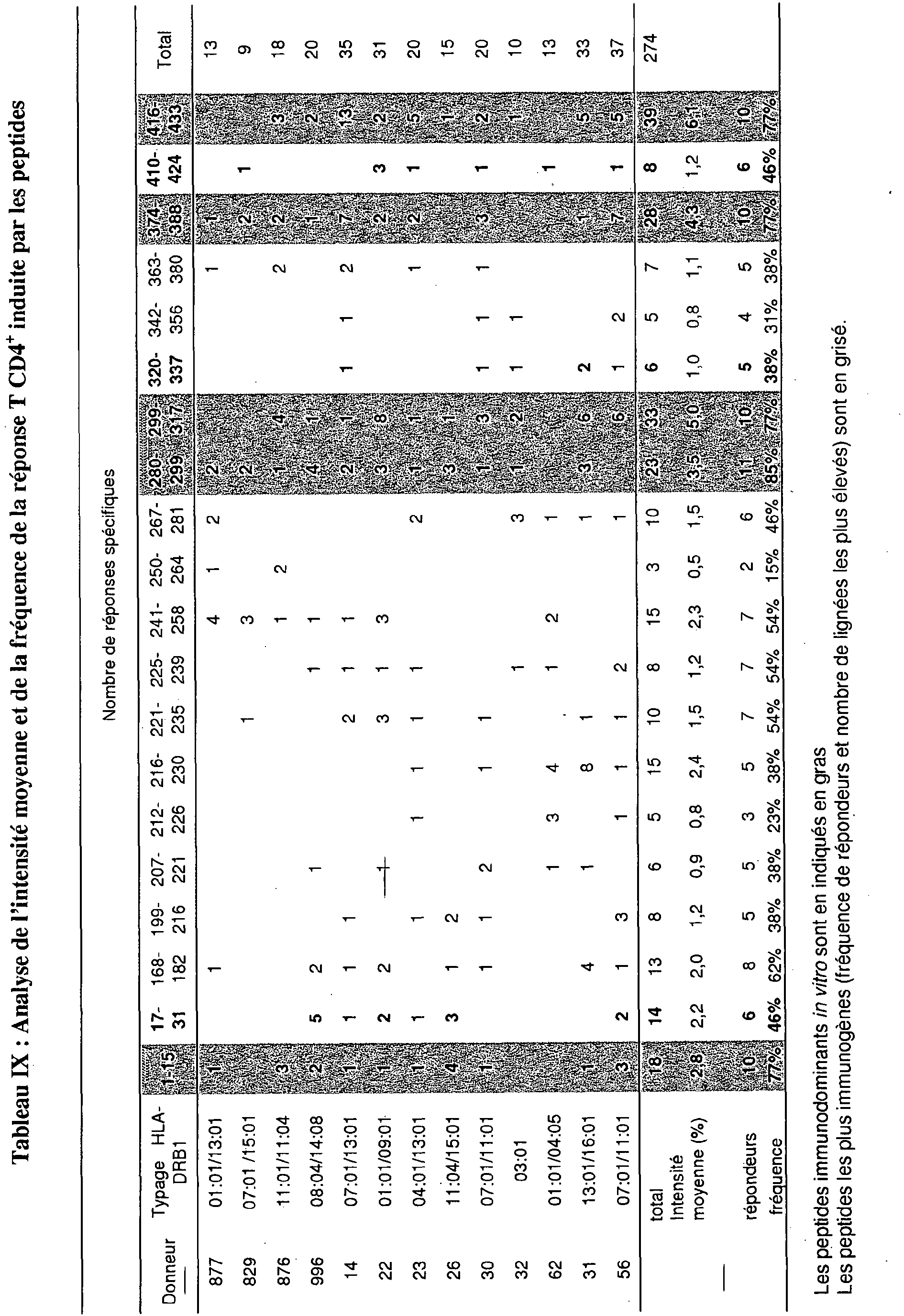

### Exemple 6 : Reconnaissance de CCNB1 par les lymphocytes T CD4⁺

De manière à évaluer la capacité des lymphocytes T CD4⁺ à reconnaître la protéine CCNB1, une partie de lignées de lymphocytes T CD4⁺ a été mise en culture en présence de cellules dendritiques autologues préalablement chargées avec CCNB1 puis leur activation a été testée par Elispot IFN-gamma. Des exemples de réponses à la protéine CCNB1 sont présentés à la Figure 2A. L'ensemble des peptides ayant généré des lymphocytes T CD4⁺ capables de reconnaitre la protéine CCNB1 est présenté à la Figure 2B. Tous les peptides n'ont pas pu être testés.

**Tableau X: Séquences d'acides aminés**

| **Nom** | **Séquence** | **SEQ ID NO :** |
|---|---|---|
| CCNB1 | | 1 |
| HA 306-318 | PKYVKQNTLKLAT | 2 |
| YKL | AAYAAAKAAALAA | 3 |
| A3 152-166 | EAEQLRAYLDGTGVE | 4 |
| MT 2-16 | AKTIAYDEEARRGLE | 5 |
| B1 21-3 6 | TERVRLVTRHIYNREE | 6 |
| LOL 191-210 | ESWGAVWRIDTPDKLTGPFT | 7 |
| E2/E168 | AGDLLAIETDKATI | 8 |
| Oxy 271-287 | EKKYFAATQFEPLAARL | 9 |
| CCNB1 1-15 | MA**L**RVTRNSKINAEN | 10 |
| CCNB1 9-23 | SK**I**NAENKAKINMAG | 11 |
| CCNB1 17-31 | AK**I**NMAGAKRVPTAP | 12 |
| CCNB1 25-39 | KR**V**PTAPAATSKPGL | 13 |
| CCNB1 37-51 | PG**L**RPRTALGDIGNK | 14 |
| CCNB1 43-57 | TA**L**GDIGNKVSEQLQ | 15 |
| CCNB1 50-64 | NK**V**SEQLQAKMPMKK | 16 |
| CCNB1 54-68 | EQ**L**QAKMPMKKEAKP | 17 |
| CCNB1 58-72 | AK**M**PMKKEAKPSATG | 18 |
| CCNB1 71-85 | TGK**V**IDKKLPKPLEK | 19 |
| CCNB1 77-91 | KK**L**PKPLEKVPMLVP | 20 |
| CCNB1 81-95 | KP**L**EKVPMLVPVPVS | 21 |
| CCNB1 87-101 | PM**L**VPVPVSEPVPEP | 22 |
| CCNB1 96-110 | EP**V**PEPEPEPEPEPV | 23 |
| CCNB1 108-122 | EP**V**KEEKLSPEPILV | 24 |
| CCNB1 113-127 | EK**L**SPEPILVDTASP | 25 |
| CCNB1 118-132 | EP**I**LVDTASPSPMET | 26 |
| CCNB1 128-142 | SP**M**ETSGCAPAEEDL | 27 |
| CCNB1 140-154 | ED**L**CQAFSDVILAVN | 28 |
| CCNB1 144-158 | QA**F**SDVILAVNDVDA | 29 |
| CCNB1 147-161 | SD**V**ILAVNDVDAEDG | 30 |
| CCNB1 151-165 | LA**V**NDVDAEDGADPN | 31 |
| CCNB1 164-178 | PN**L**CSEYVKDIYAYL | 32 |
| CCNB1 168-182 | SE**Y**VKDIYAYLRQLE | 33 |
| CCNB1 172-186 | KD**I**YAYLRQLEEEQA | 34 |
| CCNB1 179-193 | RQ**L**EEEQAVRPKYLL | 35 |
| CCNB1 185-199 | QA**V**RPKYLLGREVTG | 36 |
| CCNB1 189-203 | PK**Y**LLGREVTGNMRA | 37 |
| CCNB1 195-209 | RE**V**TGNMRAILIDWL | 38 |
| CCNB1 199-213 | GN**M**RAILIDWLVQVQ | 39 |
| CCNB1 202-216 | RA**I**LIDWLVQVQMKF | 40 |
| CCNB1 207-221 | DW**L**VQVQMKFRLLQE | 41 |
| CCNB1 212-226 | VQ**M**KFRLLQETMYMT | 42 |
| CCNB1 216-230 | FR**L**LQETMYMTVSII | 43 |
| CCNB1 221-235 | ET**M**YMTVSIIDRFMQ | 44 |
| CCNB1 225-239 | MT**V**SIIDRFMQNNCV | 45 |
| CCNB1 231-245 | DR**F**MQNNCVPKKMLQ | 46 |
| CCNB1 237-251 | NC**V**PKKMLQLVGVTA | 47 |
| CCNB1 241-255 | KK**M**LQLVGVTAMFIA | 48 |
| CCNB1 244-258 | LQ**L**VGVTAMFIASKY | 49 |
| CCNB1 250-264 | TA**M**FIASKYEEMYPP | 50 |
| CCNB1 256-270 | SK**Y**EEMYPPEIGDFA | 51 |
| CCNB1 260-274 | EM**Y**PPEIGDFAFVTD | 52 |
| CCNB1 264-278 | PE**I**GDFAFVTDNTYT | 53 |
| CCNB1 267-281 | GD**F**AFVTDNTYTKHQ | 54 |
| CCNB1 269-283 | FA**F**VTDNTYTKHQIR | 55 |
| CCNB1 275-289 | NT**Y**TKHQIRQMEMKI | 56 |
| CCNB1 280-294 | HQ**I**RQMEMKILRALN | 57 |
| CCNB1 285-299 | ME**M**KILRALNFGLGR | 58 |
| CCNB1 291-305 | RA**L**NFGLGRPLPLHF | 59 |
| CCNB1 299-313 | RP**L**PLHFLRRASKIG | 60 |
| CCNB1 303-317 | LH**F**LRRASKIGEVDV | 61 |
| CCNB1 310-324 | SK**I**GEVDVEQHTLAK | 62 |
| CCNB1 315-329 | VD**V**EQHTLAKYLMEL | 63 |
| CCNB1 320-334 | HT**L**AKYLMELTMLDY | 64 |
| CCNB1 323-337 | AK**Y**LMELTMLDYDMV | 65 |
| CCNB1 327-341 | ME**L**TMLDYDMVHFPP | 66 |
| CCNB1 332-346 | LD**Y**DMVHFPPSQIAA | 67 |
| CCNB1 338-352 | H**F**PPSQIAAGAFCLA | 68 |
| CCNB1 342-356 | SQ**I**AAGAFCLALKIL | 69 |
| CCNB1 347-361 | GA**F**CLALKILDNGEW | 70 |
| CCNB1 351-365 | LA**L**KILDNGEWTPTL | 71 |
| CCNB1 359-373 | GE**W**TPTLQHYLSYTE | 72 |
| CCNB1 363-377 | PT**L**QHYLSYTEESLL | 73 |
| CCNB1 366-380 | QH**Y**LSYTEESLLPVM | 74 |
| CCNB1 369-383 | LS**Y**TEESLLPVMQHL | 75 |
| CCNB1 374-388 | ES**L**LPVMQHLAKNVV | 76 |
| CCNB1 377-391 | LP**V**MQHLAKNWMVN | 77 |
| CCNB1 385-399 | KN**V**VMVNQGLTKHMT | 78 |
| CCNB1 392-406 | QG**L**TKHMTVKNKYAT | 79 |
| CCNB1 396-410 | KH**M**TVKNKYATSKHA | 80 |
| CCNB1 402-416 | NK**Y**ATSKHAKISTLP | 81 |
| CCNB1 410-424 | AK**I**STLPQLNSALVQ | 82 |
| CCNB1 416-430 | PQ**L**NSALVQDLAKAV | 83 |
| CCNB1 419-433 | NSA**L**VQDLAKAVAKV | 84 |
| CCNB1 199-216 | GNMRAILIDWLVQVQMKF | 85 |
| CCNB1 241-258 | KKMLQLVGVTAMFIASKY | 86 |
| CCNB1 280-299 | HQIRQMEMKILRALNFGLGR | 87 |
| CCNB1 299-317 | RPLPLHFLRRASKIGEVDV | 88 |
| CCNB1 320-337 | HTLAKYLMELTMLDYDMV | 89 |
| CCNB1 363-380 | PTLQHYLSYTEESLLPVM | 90 |
| CCNB1 416-433 | PQLNSALVQDLAKAVAKV | 91 |
| survivine 96-104 | LTLGEFLKL | 92 |
| survivine 95-104 | ELTLGEFLKL | 93 |
| survivine-2B 80-88 | AYACNTSTL | 94 |
| PADRE | KXVAAWTLKAA | 95 |
| P1 | AGYLMELCV | 96 |
| P2 | AGYLMELCM | 97 |
| P3 | AGYLMELCF | 98 |
| P4 | AGYLMELCC | 99 |
| P5 | AGYLMELCMA | 100 |
| P6 | AGYLMELCFA | 101 |
| CB9 | AKYLMELTM | 102 |
| CB10 | AKYLMELTML | 103 |
| CB9L2 | ALYLMELTM | 104 |
| CB9M2 | AMYLMELTM | 105 |
| CB204 | ILIDWLVQV | 106 |
| CB215-229 | KFRLLQETMYMTVSI | 107 |
| CB219-233 | LQETMYMTVSIIDRF | 108 |
| CB223-234 | MYMTVSIIDRFM | 109 |

## Revendications

1. Peptide isolé dérivé de la séquence de la cycline B1 humaine de SEQ ID NO : 1 et comprenant au moins un épitope T CD4⁺ immunodominant *in vitro* de la cycline B1 humaine, ledit peptide étant sélectionné dans le groupe constitué par :
a) les séquences d'au plus 30 acides aminés consécutifs de la séquence de la cycline B1 humaine de SEQ ID NO : 1 comprenant l'une des séquences SEQ ID NO : 10, 57, 58, 61, 76, 83, 84, 87, 88 et 91, et
b) les séquences de 15 à 25 acides aminés présentant au moins 85 % d'identité avec une séquence en a),
à l'exclusion de la séquence de 15 acides aminés constituée par les résidus 279 à 293 de ladite séquence SEQ ID NO : 1, et
ledit peptide en a) et b) étant capable de stimuler une réponse en lymphocytes T CD4+ humains spécifiques telle que l'intensité moyenne de la réponse *in vitro* en lymphocytes T CD4⁺ humains spécifiques dudit peptide est d'au moins 2,8 % dans un groupe d'individus humains exprimant des molécules HLA II variées incluant au moins les molécules HLA-DR1, HLA-DR3, HLA-DR4, HLA-DR7, HLA-DR11, HLA-DR13 et HLA-DR15, et la fréquence de répondeurs *in vitro* audit peptide est d'au moins 65 % dans ledit groupe d'individus humains.

2. Peptide selon la revendication 1, **caractérisé en ce que** les séquences en b) présentent au moins 95 % d'identité avec une séquence en a).

3. Peptide selon la revendication 1, sélectionné dans le groupe constitué par les séquences SEQ ID NO : 10, 57, 58, 61, 76, 83, 84, 87, 88 et 91.

4. Composition immunogène ou vaccinale, comprenant au moins un premier peptide comprenant au moins un épitope T CD4⁺ immunodominant *in vitro* de la cycline B1 humaine selon l'une quelconque des revendications 1 à 3, et un véhicule pharmaceutiquement acceptable, une substance porteuse ou un adjuvant.

5. Composition immunogène ou vaccinale selon la revendication 4, comprenant en outre au moins un second peptide comprenant au moins un épitope T CD4⁺ différent dudit épitope immunodominant *in vitro,* un épitope T CD8⁺, et/ou un épitope B, et ledit ou lesdits premier(s) et second peptides(s) étant isolés ou associés entre eux sous la forme d'un polypeptide multi-épitopique comprenant moins de 50 acides aminés consécutifs de la cycline B1 humaine de SEQ ID NO : 1.

6. Composition immunogène ou vaccinale selon la revendication 5, dans laquelle ledit second peptide est un peptide comprenant un épitope T CD4⁺ de la cycline B1, capable de se lier à au moins 6 molécules HLA II prépondérantes différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 et HLA-DP402, sélectionné dans le groupe constitué par : a) les séquences d'au plus 30 acides aminés consécutifs de la séquence de la cycline B1 humaine de SEQ ID NO : 1 comprenant l'une des séquences SEQ ID NO : 33, 39, 40, 41, 42, 43, 44, 45, 48, 49, 50, 54, 60, 69, 73, 74, 75, 85, 86 et 90, et b) les séquences de 15 à 25 acides aminés présentant au moins 85 % d'identité avec une séquence en a).

7. Composition immunogène ou vaccinale selon la revendication 6, dans laquelle ledit second peptide est sélectionné dans le groupe constitué par les séquences SEQ ID NO : 33, 39, 40, 41, 42, 43, 44, 45, 48, 49, 50, 54, 60, 69, 73, 74, 75, 85, 86 et 90.

8. Composition immunogène ou vaccinale selon la revendication 5, dans laquelle ledit second peptide est un peptide comprenant un épitope T CD4⁺ de la cycline B1, capable de se lier à 3 à 5 molécules HLA II prépondérantes différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-D.RB5, HLA-DP401 et HLA-DP402, sélectionné dans le groupe constitué par : a) les séquences d'au plus 30 acides aminés consécutifs de la séquence de la cycline B1 humaine de SEQ ID NO : 1 comprenant l'une des séquences SEQ ID NO : 25, 29, 32, 34, 36, 37, 38, 47, 53, 55, 56, 59, 66, 72 et 78, et b) les séquences de 15 à 25 acides aminés présentant au moins 85 % d'identité avec une séquence en a).

9. Composition immunogène ou vaccinale selon la revendication 8, dans laquelle ledit second peptide est sélectionné dans le groupe constitué par les séquences SEQ ID NO : 25, 29, 32, 34, 36, 37, 38, 47, 53, 55, 56, 59, 66, 72 et 78.

10. Composition immunogène ou vaccinale selon l'une quelconque des revendications 4 à 9, dans laquelle la séquence d'acides aminés du premier peptide ou du polypeptide multi-épitopique comprend au moins une modification chimique sélectionnée dans le groupe constitué par : l'acétylation du résidu d'acide aminé N-terminal et/ou l'amidation du résidu d'acide aminé C-terminal, la substitution d'un acide aminé par un acide aminé non-protéogénique, l'addition d'un groupement lipide, oligosaccharide ou polysaccharide au niveau d'une fonction réactive de la chaîne latérale d'un acide aminé, au moins une N6-acétyl-lysine, phosphosérine et/ou phosphothréonine, la modification de la liaison peptidique par une liaison du type rétro ou rétro-inverso et la fusion de la séquence d'acides aminés dudit peptide ou polypeptide avec la séquence d'acides aminés d'une protéine ou d'un polypeptide hétérologues d'intérêt, et ledit premier peptide ou polypeptide multi-épitopique comprenant ladite modification chimique comprenant au moins un épitope T CD4⁺ immunodominant *in vitro* de la cycline B1 humaine et étant capable de stimuler une réponse en lymphocytes T CD4+ humains spécifiques telle que définie à la revendication 1.

11. Polynucléotide isolé codant pour un peptide selon l'une quelconque des revendications 1 à 3 et 10 ou un polypeptide multi-épitopique tel que défini à la revendication 5 ou 10.

12. Vecteur recombinant d'expression comprenant un polynucléotide selon la revendication 11.

13. Composition immunogène ou vaccinale selon l'une quelconque des revendications 4 à 10, pour son utilisation dans l'immunothérapie prophylactique ou curative d'un cancer associé à la surexpression de la cycline B1.

## Patentansprüche

1. Isoliertes Peptid, welches von der Sequenz des humanen Cyclin B1 von SEQ ID NO:1 abgeleitet ist und wenigstens ein in-vitro immundominantes T CD4⁺-Epitop des humanen Cyclin B1 umfasst, wobei das Peptid gewählt ist aus der Gruppe, umfassend:
a) die Sequenzen mit mehr als 30 aufeinanderfolgenden Aminosäuren der Sequenz des humanen Cyclin B1 von SEQ ID NO:1, umfassend eine der Sequenzen SEQ ID NO: 10, 57, 58, 61, 76, 83, 84, 87, 88 und 91, und
b) die Sequenzen mit 15 bis 25 Aminosäuren, welche wenigstens 85% Identität mit einer Sequenz aus a) aufweisen,
unter Ausschluss der Sequenz mit 15 Aminosäuren, die durch die Reste 279 bis 293 der Sequenz SEQ ID NO 1 gebildet ist, und
wobei das Peptid aus a) und b) in der Lage ist, eine Reaktion der spezifischen humanen T CD4+-Lymphozyten zu stimulieren, sodass die mittlere Intensität der in-vitro-Reaktion der spezifischen humanen T-CD4+-Lymphozyten des Peptids in einer Gruppe menschlicher Individuen, die verschiedenartige HLA 11-Moleküle, wenigstens einschließlich der Moleküle HLA-DR1, HLA-DR3, HLA-DR4, HLA-DR7, HLA-DR11, HLA-DR13 und HLA-DR15, exprimieren bei wenigstens 2,8 % liegt und die Häufigkeit von in-vitro-Respondern auf das Peptid in der Gruppe menschlicher Individuen bei wenigstens 65 % liegt.

2. Peptid nach Anspruch 1, **dadurch gekennzeichnet, dass** die Sequenzen aus b) wenigstens 95 % Identität mit einer Sequenz aus a) aufweisen.

3. Peptid nach Anspruch 1, gewählt aus der Gruppe, umfassend die Sequenzen SEQ ID NO: 10, 57, 58, 61, 76, 83, 84, 87, 88 und 91.

4. Immunogene Zusammensetzung oder Impfstoffzusammensetzung, welche wenigstens ein erstes Peptid umfasst, das wenigstens ein in-vitro immundominantes T-CD4⁺-Epitop des humanen Cyclin B1 nach einem der Ansprüche 1 bis 3 aufweist, sowie einen pharmazeutisch akzeptablen Träger, eine Trägersubstanz oder einen Hilfsstoff.

5. Immunogene Zusammensetzung oder Impfstoffzusammensetzung nach Anspruch 4, welche ferner wenigstens ein zweites Peptid umfasst, das wenigstens ein von dem in-vitro immundominanten Epitop verschiedenes in-vitro immundominantes T-CD4⁺-Epitop, ein T-CD8⁺-Epitop und/oder ein B-Epitop aufweist, und wobei das erste/die ersten und das zweite/die zweiten Peptide isoliert oder als multi-epitopisches Polypeptid miteinander verbunden sind und weniger als 50 aufeinanderfolgende Aminosäuren des humanen Cyclin B1 von SEQ ID NO:1 umfassen.

6. Immunogene Zusammensetzung oder Impfstoffzusammensetzung nach Anspruch 5, wobei das zweite Peptid ein Peptid ist, welches ein T-CD4⁺-Epitop des Cyclin B1 umfasst, welches in der Lage ist, eine Bindung mit wenigstens 6 vorherrschenden unterschiedlichen HLA II-Molekülen einzugehen, die gewählt sind aus den Molekülen HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 und HLA-DP402, gewählt aus der Gruppe, umfassend: a) die Sequenzen mit mehr als 30 aufeinanderfolgenden Aminosäuren der Sequenz des humanen Cyclin B1 von SEQ ID NO:1, umfassend eine der Sequenzen SEQ ID NO: 33, 39, 40, 41, 42, 43, 44, 45, 48, 49, 50, 54, 60, 69, 73, 74, 75, 85, 86 und 90, und b) die Sequenzen mit 15 bis 25 Aminosäuren, welche wenigstens 85% Identität mit einer Sequenz aus a) aufweisen.

7. Immunogene Zusammensetzung oder Impfstoffzusammensetzung nach Anspruch 6, wobei das zweite Peptid gewählt ist aus der Gruppe, umfassend die Sequenzen SEQ ID NO: 33, 39, 40, 41, 42, 43, 44, 45, 48, 49, 50, 54, 60, 69, 73, 74, 75, 85, 86 und 90.

8. Immunogene Zusammensetzung oder Impfstoffzusammensetzung nach Anspruch 5, wobei das zweite Peptid ein Peptid ist, welches ein T-CD4⁺-Epitop des Cyclin B1 umfasst, welches in der Lage ist, eine Bindung mit 3 bis 5 vorherrschenden unterschiedlichen HLA II-Molekülen einzugehen, die gewählt sind aus den Molekülen HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 und HLA-DP402, gewählt aus der Gruppe, umfassend: a) die Sequenzen mit mehr als 30 aufeinanderfolgenden Aminosäuren der Sequenz des humanen Cyclin B1 von SEQ ID NO:1, umfassend eine der Sequenzen SEQ ID NO: 25, 29, 32, 34, 36, 37, 38, 47, 53, 55, 56, 59, 66, 72 und 78, und b) die Sequenzen mit 15 bis 25 Aminosäuren, welche wenigstens 85% Identität mit einer Sequenz aus a) aufweisen.

9. Immunogene Zusammensetzung oder Impfstoffzusammensetzung nach Anspruch 8, wobei das zweite Peptid gewählt ist aus der Gruppe, umfassend die Sequenzen SEQ ID NO: 25, 29, 32, 34, 36, 37, 38, 47, 53, 55, 56, 59, 66, 72 und 78.

10. Immunogene Zusammensetzung oder Impfstoffzusammensetzung nach einem der Ansprüche 4 bis 9, wobei die Aminosäuresequenz des ersten Peptids oder des multiepitopischen Polypeptids wenigstens eine chemische Modifizierung umfasst, welche gewählt ist aus der Gruppe, umfassend: Acetylierung des N-terminalen Aminosäurerests und/oder Amidierung des C-terminalen Aminosäurerests, Substitution einer Aminosäure durch eine nicht-proteinogene Aminosäure, Hinzufügen einer Lipid-, Oligosaccharid- oder Polysaccharid-Gruppe an einer reaktionsfähigen Funktion der Seitenkette einer Aminosäure, wenigstens ein N6-Acetyl-Lysin, Phosphoserin und/oder Phosphothreonin, die Modifizierung der Peptidbindung durch eine Bindung vom Retro- oder Retro-Invers-Typ und die Fusion der Aminosäuresequenz des Peptids oder Polypeptids mit der Aminosäuresequenz eines Proteins oder eines heterologen Polypeptids von Interesse, und wobei das erste Peptid oder multiepitopische Polypetid mit dieser chemischen Modifizierung wenigstens ein in-vitro immundominantes CD4⁺-Epitop des humanen Cyclin B1 umfasst und in der Lage ist, eine Reaktion der spezifischen humanen T-CD4+-Lymphozyten nach Anspruch 1 zu stimulieren.

11. Isoliertes Polynukleotid, welches ein Peptid nach einem der Ansprüche 1 bis 3 und 10 oder ein multi-epitopisches Polypeptid nach einem der Ansprüche 5 oder 10 kodiert.

12. Rekombinanter Expressionsvektor, welcher ein Polynukleotid nach Anspruch 11 umfasst.

13. Immunogene Zusammensetzung oder Impfstoffzusammensetzung nach einem der Ansprüche 4 bis 10, zur Verwendung in der prophylaktischen oder kurativen Immuntherapie einer mit einer Überexpression des Cyclin B1 assoziierten Krebserkrankung.

## Claims

1. Isolated peptide derived from the human cyclin B1 sequence of SEQ ID NO. 1 and which comprises at least one human cyclin B1 CD4⁺ T epitope which is immunodominant *in vitro,* said peptide being selected from the group consisting of:
a) the sequences of up to 30 consecutive amino acids of the human cyclin B1 sequence of SEQ ID NO. 1 comprising one of the sequences of SEQ ID NO: 10, 57, 58, 61, 76, 77, 83, 84, 87, 88 and 91, and
b) the sequences of 15 to 25 amino acids having at least 85 % identity with a sequence in a),
with the exclusion of the sequence of 15 amino acids consisting of residues 279 to 293 of said sequence SEQ ID NO. 1, and
said peptide in a) and b) being capable of stimulating a specific human CD4⁺ T lymphocyte response wherein the average strength of the *in vitro* response of human CD4⁺ T lymphocytes specific for said peptide is at least 2.8% in a group of human individuals expressing varied HLA II molecules including at least the HLA-DR1, HLA-DR3, HLA-DR4, HLA-DR7, HLA-DR11, HLA-DR13 and HLA-DR15 molecules, and the frequency of responders *in vitro* to said peptide is at least 65% in said group of human individuals.

2. The peptide as claimed in claim 1, **characterized in that** the sequences in b) have at least 95 % identity with a sequence in a).

3. The peptide as claimed in claim 1, selected from the group consisting of the sequences SEQ ID NO: 10, 57, 58, 61, 76, 83, 84, 87, 88 and 91.

4. Immunogenic or vaccine composition, comprising at least one first peptide comprising at least one cyclin B1 CD4⁺ T epitope which is immunodominant *in vitro* as claimed in any one of claims 1 to 3, and a pharmaceutically acceptable vehicle, a carrier substance or an adjuvant.

5. Immunogenic or vaccine composition as claimed in claim 4, further comprising at least one second peptide comprising at least one CD4⁺ T epitope which is different from said epitope which is immunodominant *in vitro,* one CD8⁺ T epitope, and/or one B epitope, and said first(s) and second(s) peptide(s) being isolated or linked to one another in the form of a multi-epitope polypeptide comprising less than 50 consecutive amino acids of human cyclin B1 of SEQ ID NO. 1.

6. Immunogenic or vaccine composition as claimed in claim 5, wherein said second peptide is a peptide comprising a cyclin B1 CD4⁺ T epitope, capable of binding to at least 6 different predominant HLA II molecules chosen from the HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 and HLA-DP402 molecules, selected from the group consisting of: a) the sequences of up to 30 consecutive amino acids of the human cyclin B1 sequence of SEQ ID NO: 1 comprising one of the sequences of SEQ ID NO: 33, 39, 40, 41, 42, 43, 44, 45, 48, 49, 50, 54, 60, 69, 73, 74, 75, 85, 86 and 90, and b) the sequences of 15 to 25 amino acids having at least 85% identity with a sequence in a).

7. Immunogenic or vaccine composition as claimed in claim 6, wherein said second peptide is selected from the group consisting of the sequences SEQ ID NO: 33, 39, 40, 41, 42, 43, 44, 45, 48, 49, 50, 54, 60, 69, 73, 74, 75, 85, 86 and 90.

8. Immunogenic or vaccine composition as claimed in claim 5, wherein said second peptide is a peptide comprising a cyclin B1 CD4⁺ T epitope, capable of binding to 3 to 5 different predominant HLA II molecules chosen from the HLA-DR1, HLA-DR3, HLA-DR4, HLA-DR7, HLA-DR11, HLA-DR13, HLA-DR15, HLA-DRB3, HLA-DRB4, HLA-DRB5, HLA-DP401 and HLA-DP402 molecules, selected from the group consisting of: a) the sequences of up to 30 consecutive amino acids of the human cyclin B1 sequence of SEQ ID NO: 1 comprising one of the sequences SEQ ID NO: 25, 29, 32, 34, 36, 37, 38, 47, 53, 55, 56, 59, 66, 72 and 78, and b) the sequences of 15 to 25 amino acids having at least 85% identity with a sequence in a).

9. Immunogenic or vaccine composition as claimed in claim 8, wherein said second peptide is selected from the group consisting of the sequences SEQ ID NO: 25, 29, 32, 34, 36, 37, 38, 47, 53, 55, 56, 59, 66, 72 and 78.

10. Immunogenic or vaccine composition as claimed in any one of claims 4 to 9, wherein the amino acid sequence of said first peptide or of said multi-epitope peptide comprises at least one chemical modification selected from the group consisting of: acetylation of the N-terminal residue and/or amidation of the C-terminal residue, substitution of an amino acid with a non-proteinogenic amino acid, addition of a lipid, oligosaccharide or polysaccharide group on a reactive function of the lateral chain of an amino acid, at least one N6-acetyl-lysine, phosphoserine and/or phosphothreonine, modification of the peptide bond with a bond of the retro or retro-inverso type and fusion of the amino acid sequence of said peptide or polypeptide with the amino acid sequence of an heterologous protein or peptide of interest, and said first peptide or said polypeptide comprising said modification comprising at least one human cyclin B1 CD4⁺ T epitope which is immunodominant *in vitro* and being capable of stimulating a specific human CD4⁺ T lymphocyte response as defined in claim 1.

11. Isolated polynucleotide encoding a peptide as claimed in any one of claims 1 to 3 and 10 or a multi-epitope polypeptide as claimed in claim 5 or 10.

12. Recombinant expression vector comprising a polynucleotide as claimed in claim 11.

13. Immunogenic or vaccine composition as claimed in any one of claims 4 to 10, for use in the prophylactic or curative immunotherapy of a cancer associated with the overexpression of cyclin B1.


### SEQUENCE LISTING

<110> COMMISSARIAT A L'ENERGIE ATOMIQUE ET AUX ENERGIES ALTERNATIVES
<120> PEPTIDES IMMUNOGENES DE L'ANTIGENE TUMORAL CYCLINE B1
<130> 263PCT571
<160> 109
<170> PatentIn version 3.5
<210> 1
   <211> 433
   <212> PRT
   <213> Homo sapiens
<400> 1
   Met Ala Leu Arg Val Thr Arg Asn Ser Lys Ile Asn Ala Glu Asn Lys
   1               5                   10                  15
   Ala Lys Ile Asn Met Ala Gly Ala Lys Arg Val Pro Thr Ala Pro Ala
               20                  25                  30
   Ala Thr Ser Lys Pro Gly Leu Arg Pro Arg Thr Ala Leu Gly Asp Ile
           35                  40                  45
   Gly Asn Lys Val Ser Glu Gln Leu Gln Ala Lys Met Pro Met Lys Lys
       50                  55                  60
   Glu Ala Lys Pro Ser Ala Thr Gly Lys Val Ile Asp Lys Lys Leu Pro
   65                  70                  75                  80
   Lys Pro Leu Glu Lys Val Pro Met Leu Val Pro Val Pro Val Ser Glu
                   85                  90                  95
   Pro Val Pro Glu Pro Glu Pro Glu Pro Glu Pro Glu Pro Val Lys Glu
               100                 105                 110
   Glu Lys Leu Ser Pro Glu Pro Ile Leu Val Asp Thr Ala Ser Pro Ser
           115                 120                 125
   Pro Met Glu Thr Ser Gly Cys Ala Pro Ala Glu Glu Asp Leu Cys Gln
       130                 135                 140
   Ala Phe Ser Asp Val Ile Leu Ala Val Asn Asp Val Asp Ala Glu Asp
   145                 150                 155                 160
   Gly Ala Asp Pro Asn Leu Cys Ser Glu Tyr Val Lys Asp Ile Tyr Ala
                   165                 170                 175
   Tyr Leu Arg Gln Leu Glu Glu Glu Gln Ala Val Arg Pro Lys Tyr Leu
               180                 185                 190
   Leu Gly Arg Glu Val Thr Gly Asn Met Arg Ala Ile Leu Ile Asp Trp
           195                 200                 205
   Leu Val Gln Val Gln Met Lys Phe Arg Leu Leu Gln Glu Thr Met Tyr
       210                 215                 220
   Met Thr Val Ser Ile Ile Asp Arg Phe Met Gln Asn Asn Cys Val Pro
   225                 230                 235                 240
   Lys Lys Met Leu Gln Leu Val Gly Val Thr Ala Met Phe Ile Ala Ser
                   245                 250                 255
   Lys Tyr Glu Glu Met Tyr Pro Pro Glu Ile Gly Asp Phe Ala Phe Val
               260                 265                 270
   Thr Asp Asn Thr Tyr Thr Lys His Gln Ile Arg Gln Met Glu Met Lys
           275                 280                 285
   Ile Leu Arg Ala Leu Asn Phe Gly Leu Gly Arg Pro Leu Pro Leu His
       290                 295                 300
   Phe Leu Arg Arg Ala Ser Lys Ile Gly Glu Val Asp Val Glu Gln His
   305                 310                 315                 320
   Thr Leu Ala Lys Tyr Leu Met Glu Leu Thr Met Leu Asp Tyr Asp Met
                   325                 330                 335
   Val His Phe Pro Pro Ser Gln Ile Ala Ala Gly Ala Phe Cys Leu Ala
               340                 345                 350
   Leu Lys Ile Leu Asp Asn Gly Glu Trp Thr Pro Thr Leu Gln His Tyr
           355                 360                 365
   Leu Ser Tyr Thr Glu Glu Ser Leu Leu Pro Val Met Gln His Leu Ala
       370                 375                 380
   Lys Asn Val Val Met Val Asn Gln Gly Leu Thr Lys His Met Thr Val
   385                 390                 395                 400
   Lys Asn Lys Tyr Ala Thr Ser Lys His Ala Lys Ile Ser Thr Leu Pro
                   405                 410                 415
   Gln Leu Asn Ser Ala Leu Val Gln Asp Leu Ala Lys Ala Val Ala Lys
               420                 425                 430
   Val
<210> 2
   <211> 13
   <212> PRT
   <213> artificial sequence
<220>
   <223> synthetic peptide HA 306-318
<400> 2
   Pro Lys Tyr Val Lys Gln Asn Thr Leu Lys Leu Ala Thr
   1               5                   10
<210> 3
   <211> 13
   <212> PRT
   <213> artificial sequence
<220>
   <223> synthetic peptide YKL
<400> 3
   Ala Ala Tyr Ala Ala Ala Lys Ala Ala Ala Leu Ala Ala
   1               5                   10
<210> 4
   <211> 15
   <212> PRT
   <213> artificial sequence
<220>
   <223> synthetic peptide A3 152-166
<400> 4
   Glu Ala Glu Gln Leu Arg Ala Tyr Leu Asp Gly Thr Gly Val Glu
   1               5                   10                  15
<210> 5
   <211> 15
   <212> PRT
   <213> artificial sequence
<220>
   <223> synthetic peptide MT 2-16
<400> 5
   Ala Lys Thr Ile Ala Tyr Asp Glu Glu Ala Arg Arg Gly Leu Glu
   1               5                   10                  15
<210> 6
   <211> 16
   <212> PRT
   <213> artificial sequence
<220>
   <223> synthetic peptide B1 21-36
<400> 6
   Thr Glu Arg Val Arg Leu Val Thr Arg His Ile Tyr Asn Arg Glu Glu
   1               5                   10                  15
<210> 7
   <211> 20
   <212> PRT
   <213> artificial sequence
<220>
   <223> synthetic peptide LOL 191-210
<400> 7
   Glu Ser Trp Gly Ala Val Trp Arg Ile Asp Thr Pro Asp Lys Leu Thr
   1               5                   10                  15
   Gly Pro Phe Thr
               20
<210> 8
   <211> 14
   <212> PRT
   <213> artificial sequece
<220>
   <223> synthetic peptide E2/E168
<400> 8
   Ala Gly Asp Leu Leu Ala Ile Glu Thr Asp Lys Ala Thr Ile
   1               5                   10
<210> 9
   <211> 17
   <212> PRT
   <213> artificial sequence
<220>
   <223> synthetic peptide Oxy 271-287
<400> 9
<210> 10
   <211> 15
   <212> PRT
   <213> artificial sequence
<220>
   <223> synthetic peptide CCNB1 1-15
<400> 10
   Met Ala Leu Arg Val Thr Arg Asn Ser Lys Ile Asn Ala Glu Asn
   1               5                   10                  15
<210> 11
   <211> 15
   <212> PRT
   <213> artificial sequence
<220>
   <223> synthetic peptide CCNB1 9-23
<400> 11
   Ser Lys Ile Asn Ala Glu Asn Lys Ala Lys Ile Asn Met Ala Gly
   1               5                   10                  15
<210> 12
   <211> 15
   <212> PRT
   <213> artificial sequence
<220>
   <223> synthetic peptide CCNB1 17-31
<400> 12
   Ala Lys Ile Asn Met Ala Gly Ala Lys Arg Val Pro Thr Ala Pro
   1               5                   10                  15
<210> 13
   <211> 15
   <212> PRT
   <213> artificial sequence
<220>
   <223> synthetic peptide CCNB1 25-39
<400> 13
   Lys Arg Val Pro Thr Ala Pro Ala Ala Thr Ser Lys Pro Gly Leu
   1               5                   10                  15
<210> 14
   <211> 15
   <212> PRT
   <213> artificial sequence
<220>
   <223> synthetic peptide CCNB1 37-51
<400> 14
   Pro Gly Leu Arg Pro Arg Thr Ala Leu Gly Asp Ile Gly Asn Lys
   1 5 10 15
<210> 15
   <211> 15
   <212> PRT
   <213> artificial sequence
<220>
   <223> synthetic peptide CCNB1 43-57
<400> 15
   Thr Ala Leu Gly Asp Ile Gly Asn Lys Val Ser Glu Gln Leu Gln
   1 5 10 15
<210> 16
   <211> 15
   <212> PRT
   <213> artificial sequence
<220>
   <223> synthetic peptide CCNB1 50-64
<400> 16
   Asn Lys Val Ser Glu Gln Leu Gln Ala Lys Met Pro Met Lys Lys
   1 5 10 15
<210> 17
   <211> 15
   <212> PRT
   <213> artificial sequence
<220>
   <223> synthetic peptide CCNB1 54-68
<400> 17
   Glu Gln Leu Gln Ala Lys Met Pro Met Lys Lys Glu Ala Lys Pro
   1 5 10 15
<210> 18
   <211> 15
   <212> PRT
   <213> artificial sequence
<220>
   <223> synthetic peptide CCNB1 58-72
<400> 18
   Ala Lys Met Pro Met Lys Lys Glu Ala Lys Pro Ser Ala Thr Gly
   1 5 10 15
<210> 19
   <211> 15
   <212> PRT
   <213> artificial sequence
<220>
   <223> synthetic peptide CCNB1 71-85
<400> 19
   Thr Gly Lys Val Ile Asp Lys Lys Leu Pro Lys Pro Leu Glu Lys
   1 5 10 15
<210> 20
   <211> 15
   <212> PRT
   <213> artificial sequence
<220>
   <223> synthetic peptide CCNB1 77-91
<400> 20
   Lys Lys Leu Pro Lys Pro Leu Glu Lys Val Pro Met Leu Val Pro
   1 5 10 15
<210> 21
   <211> 15
   <212> PRT
   <213> artificial sequence
<220>
   <223> synthetic peptide CCNB1 81-95
<400> 21
   Lys Pro Leu Glu Lys Val Pro Met Leu Val Pro Val Pro Val Ser
   1 5 10 15
<210> 22
   <211> 15
   <212> PRT
   <213> artificial sequence
<220>
   <223> synthetic peptide CCNB1 87-101
<400> 22
   Pro Met Leu Val Pro Val Pro Val Ser Glu Pro Val Pro Glu Pro
   1 5 10 15
<210> 23
   <211> 15
   <212> PRT
   <213> artificial sequence
<220>
   <223> synthetic peptide CCNB1 96-110
<400> 23
   Glu Pro Val Pro Glu Pro Glu Pro Glu Pro Glu Pro Glu Pro Val
   1               5                   10                  15
<210> 24
   <211> 15
   <212> PRT
   <213> artificial sequence
<220>
   <223> synthetic peptide CCNB1 108-122
<400> 24
   Glu Pro Val Lys Glu Glu Lys Leu Ser Pro Glu Pro Ile Leu Val
   1               5                   10                  15
<210> 25
   <211> 15
   <212> PRT
   <213> artificial sequence
<220>
   <223> synthetic peptide CCNB1 113-127
<400> 25
   Glu Lys Leu Ser Pro Glu Pro Ile Leu Val Asp Thr Ala Ser Pro
   1               5                   10                  15
<210> 26
   <211> 15
   <212> PRT
   <213> artificial sequence
<220>
   <223> synthetic peptide CCNB1 118-132
<400> 26
   Glu Pro Ile Leu Val Asp Thr Ala Ser Pro Ser Pro Met Glu Thr
   1               5                   10                  15
<210> 27
   <211> 15
   <212> PRT
   <213> artificial sequence
<220>
   <223> synthetic peptide CCNB1 128-142
<400> 27
   Ser Pro Met Glu Thr Ser Gly Cys Ala Pro Ala Glu Glu Asp Leu
   1               5                   10                  15
<210> 28
   <211> 15
   <212> PRT
   <213> artificial sequence
<220>
   <223> synthetic peptide CCNB1 140-154
<400> 28
   Glu Asp Leu Cys Gln Ala Phe Ser Asp Val Ile Leu Ala Val Asn
   1               5                   10                  15
<210> 29
   <211> 15
   <212> PRT
   <213> artificial sequence
<220>
   <223> synthetic peptide CCNB1 144-158
<400> 29
   Gln Ala Phe Ser Asp Val Ile Leu Ala Val Asn Asp Val Asp Ala
   1               5                   10                  15
<210> 30
   <211> 15
   <212> PRT
   <213> artificial sequence
<220>
   <223> synthetic peptide CCNB1 147-161
<400> 30
   Ser Asp Val Ile Leu Ala Val Asn Asp Val Asp Ala Glu Asp Gly
   1               5                   10                  15
<210> 31
   <211> 15
   <212> PRT
   <213> artificial sequence
<220>
   <223> synthetic peptide CCNB1 151-165
<400> 31
   Leu Ala Val Asn Asp Val Asp Ala Glu Asp Gly Ala Asp Pro Asn
   1               5                   10                  15
<210> 32
   <211> 15
   <212> PRT
   <213> artificial sequence
<220>
   <223> synthetic peptide CCNB1 164-178
<400> 32
   Pro Asn Leu Cys Ser Glu Tyr Val Lys Asp Ile Tyr Ala Tyr Leu
   1 5 10 15
<210> 33
   <211> 15
   <212> PRT
   <213> artificial sequence
<220>
   <223> synthetic peptide CCNB1 168-182
<400> 33
   Ser Glu Tyr Val Lys Asp Ile Tyr Ala Tyr Leu Arg Gln Leu Glu
   1 5 10 15
<210> 34
   <211> 15
   <212> PRT
   <213> artificial sequence
<220>
   <223> synthetic peptide CCNB1 172-186
<400> 34
   Lys Asp Ile Tyr Ala Tyr Leu Arg Gln Leu Glu Glu Glu Gln Ala
   1 5 10 15
<210> 35
   <211> 15
   <212> PRT
   <213> artificial sequence
<220>
   <223> synthetic peptide CCNB1 179-193
<400> 35
   Arg Gln Leu Glu Glu Glu Gln Ala Val Arg Pro Lys Tyr Leu Leu
   1 5 10 15
<210> 36
   <211> 15
   <212> PRT
   <213> artificial sequence
<220>
   <223> synthetic peptide CCNB1 185-199
<400> 36
   Gln Ala Val Arg Pro Lys Tyr Leu Leu Gly Arg Glu Val Thr Gly
   1 5 10 15
<210> 37
   <211> 15
   <212> PRT
   <213> artificial sequence
<220>
   <223> synthetic peptide CCNB1 189-203
<400> 37
   Pro Lys Tyr Leu Leu Gly Arg Glu Val Thr Gly Asn Met Arg Ala
   1               5                   10                  15
<210> 38
   <211> 15
   <212> PRT
   <213> artificial sequence
<220>
   <223> synthetic peptide CCNB1 195-209
<400> 38
   Arg Glu Val Thr Gly Asn Met Arg Ala Ile Leu Ile Asp Trp Leu
   1               5                   10                  15
<210> 39
   <211> 15
   <212> PRT
   <213> artificial sequence
<220>
   <223> synthetic peptide CCNB1 199-213
<400> 39
   Gly Asn Met Arg Ala Ile Leu Ile Asp Trp Leu Val Gln Val Gln
   1               5                   10                  15
<210> 40
   <211> 15
   <212> PRT
   <213> artificial sequence
<220>
   <223> synthetic peptide CCNB1 202-216
<400> 40
   Arg Ala Ile Leu Ile Asp Trp Leu Val Gln Val Gln Met Lys Phe
   1               5                   10                  15
<210> 41
   <211> 15
   <212> PRT
   <213> artificial sequence
<220>
   <223> synthetic peptide CCNB1 207-221
<400> 41
   Asp Trp Leu Val Gln Val Gln Met Lys Phe Arg Leu Leu Gln Glu
   1 5 10 15
<210> 42
   <211> 15
   <212> PRT
   <213> artificial sequence
<220>
   <223> synthetic peptide CCNB1 212-226
<400> 42
   Val Gln Met Lys Phe Arg Leu Leu Gln Glu Thr Met Tyr Met Thr
   1 5 10 15
<210> 43
   <211> 15
   <212> PRT
   <213> artificial sequence
<220>
   <223> synthetic peptide CCNB1 216-230
<400> 43
   Phe Arg Leu Leu Gln Glu Thr Met Tyr Met Thr Val Ser Ile Ile
   1 5 10 15
<210> 44
   <211> 15
   <212> PRT
   <213> artificial sequence
<220>
   <223> synthetic peptide CCNB1 221-235
<400> 44
   Glu Thr Met Tyr Met Thr Val Ser Ile Ile Asp Arg Phe Met Gln
   1 5 10 15
<210> 45
   <211> 15
   <212> PRT
   <213> artificial sequence
<220>
   <223> synthetic peptide CCNB1 225-239
<400> 45
   Met Thr Val Ser Ile Ile Asp Arg Phe Met Gln Asn Asn Cys Val
   1 5 10 15
<210> 46
   <211> 15
   <212> PRT
   <213> artificial sequence
<220>
   <223> synthetic peptide CCNB1 231-245
<400> 46
   Asp Arg Phe Met Gln Asn Asn Cys Val Pro Lys Lys Met Leu Gln
   1               5                   10                  15
<210> 47
   <211> 15
   <212> PRT
   <213> artificial sequence
<220>
   <223> synthetic peptide CCNB1 237-251
<400> 47
   Asn Cys Val Pro Lys Lys Met Leu Gln Leu Val Gly Val Thr Ala
   1               5                   10                  15
<210> 48
   <211> 15
   <212> PRT
   <213> artificial sequence
<220>
   <223> synthetic peptide CCNB1 241-255
<400> 48
   Lys Lys Met Leu Gln Leu Val Gly Val Thr Ala Met Phe Ile Ala
   1               5                   10                  15
<210> 49
   <211> 15
   <212> PRT
   <213> artificial sequence
<220>
   <223> synthetic peptide CCNB1 244-258
<400> 49
   Leu Gln Leu Val Gly Val Thr Ala Met Phe Ile Ala Ser Lys Tyr
   1               5                   10                  15
<210> 50
   <211> 15
   <212> PRT
   <213> artificial sequence
<220>
   <223> synthetic peptide CCNB1 250-264
<400> 50
   Thr Ala Met Phe Ile Ala Ser Lys Tyr Glu Glu Met Tyr Pro Pro
   1               5                   10                  15
<210> 51
   <211> 15
   <212> PRT
   <213> artificial sequence
<220>
   <223> synthetic peptide CCNB1 256-270
<400> 51
   Ser Lys Tyr Glu Glu Met Tyr Pro Pro Glu Ile Gly Asp Phe Ala
   1               5                   10                  15
<210> 52
   <211> 15
   <212> PRT
   <213> artificial sequence
<220>
   <223> synthetic peptide CCNB1 260-274
<400> 52
   Glu Met Tyr Pro Pro Glu Ile Gly Asp Phe Ala Phe Val Thr Asp
   1               5                   10                  15
<210> 53
   <211> 15
   <212> PRT
   <213> artificial sequence
<220>
   <223> synthetic peptide CCNB1 264-278
<400> 53
   Pro Glu Ile Gly Asp Phe Ala Phe Val Thr Asp Asn Thr Tyr Thr
   1               5                   10                  15
<210> 54
   <211> 15
   <212> PRT
   <213> artificial sequence
<220>
   <223> synthetic peptide CCNB1 267-281
<400> 54
   Gly Asp Phe Ala Phe Val Thr Asp Asn Thr Tyr Thr Lys His Gln
   1               5                   10                  15
<210> 55
   <211> 15
   <212> PRT
   <213> artificial sequence
<220>
   <223> synthetic peptide CCNB1 269-283
<400> 55
   Phe Ala Phe Val Thr Asp Asn Thr Tyr Thr Lys His Gln Ile Arg
   1               5                   10                  15
<210> 56
   <211> 15
   <212> PRT
   <213> artificial sequence
<220>
   <223> synthetic peptide CCNB1 275-289
<400> 56
   Asn Thr Tyr Thr Lys His Gln Ile Arg Gln Met Glu Met Lys Ile
   1               5                   10                  15
<210> 57
   <211> 15
   <212> PRT
   <213> artificial sequence
<220>
   <223> synthetic peptide CCNB1 280-294
<400> 57
   His Gln Ile Arg Gln Met Glu Met Lys Ile Leu Arg Ala Leu Asn
   1               5                   10                  15
<210> 58
   <211> 15
   <212> PRT
   <213> artificial sequence
<220>
   <223> synthetic peptide CCNB1 285-299
<400> 58
   Met Glu Met Lys Ile Leu Arg Ala Leu Asn Phe Gly Leu Gly Arg
   1               5                   10                  15
<210> 59
   <211> 15
   <212> PRT
   <213> artificial sequence
<220>
   <223> synthetic peptide CCNB1 291-305
<400> 59
   Arg Ala Leu Asn Phe Gly Leu Gly Arg Pro Leu Pro Leu His Phe
   1               5                   10                  15
<210> 60
   <211> 15
   <212> PRT
   <213> artificial sequence
<220>
   <223> synthetic peptide CCNB1 299-313
<400> 60
   Arg Pro Leu Pro Leu His Phe Leu Arg Arg Ala Ser Lys Ile Gly
   1               5                   10                  15
<210> 61
   <211> 15
   <212> PRT
   <213> artificial sequence
<220>
   <223> synthetic peptide CCNB1 303-317
<400> 61
   Leu His Phe Leu Arg Arg Ala Ser Lys Ile Gly Glu Val Asp Val
   1               5                   10                  15
<210> 62
   <211> 15
   <212> PRT
   <213> artificial sequence
<220>
   <223> synthetic peptide CCNB1 310-324
<400> 62
   Ser Lys Ile Gly Glu Val Asp Val Glu Gln His Thr Leu Ala Lys
   1               5                   10                  15
<210> 63
   <211> 15
   <212> PRT
   <213> artificial sequence
<220>
   <223> synthetic peptide CCNB1 315-329
<400> 63
   Val Asp Val Glu Gln His Thr Leu Ala Lys Tyr Leu Met Glu Leu
   1               5                   10                  15
<210> 64
   <211> 15
   <212> PRT
   <213> artificial sequence
<220>
   <223> synthetic peptide CCNB1 320-334
<400> 64
   His Thr Leu Ala Lys Tyr Leu Met Glu Leu Thr Met Leu Asp Tyr
   1 5 10 15
<210> 65
   <211> 15
   <212> PRT
   <213> artificial sequence
<220>
   <223> synthetic peptide CCNB1 323-337
<400> 65
   Ala Lys Tyr Leu Met Glu Leu Thr Met Leu Asp Tyr Asp Met Val
   1 5 10 15
<210> 66
   <211> 15
   <212> PRT
   <213> artificial sequence
<220>
   <223> synthetic peptide CCNB1 327-341
<400> 66
   Met Glu Leu Thr Met Leu Asp Tyr Asp Met Val His Phe Pro Pro
   1 5 10 15
<210> 67
   <211> 15
   <212> PRT
   <213> artificial sequence
<220>
   <223> synthetic peptide CCNB1 332-346
<400> 67
   Leu Asp Tyr Asp Met Val His Phe Pro Pro Ser Gln Ile Ala Ala
   1 5 10 15
<210> 68
   <211> 15
   <212> PRT
   <213> artificial sequence
<220>
   <223> synthetic peptide CCNB1 338-352
<400> 68
   His Phe Pro Pro Ser Gln Ile Ala Ala Gly Ala Phe Cys Leu Ala
   1 5 10 15
<210> 69
   <211> 15
   <212> PRT
   <213> artificial sequence
<220>
   <223> synthetic peptide CCNB1 342-356
<400> 69
   Ser Gln Ile Ala Ala Gly Ala Phe Cys Leu Ala Leu Lys Ile Leu
   1 5 10 15
<210> 70
   <211> 15
   <212> PRT
   <213> artificial sequence
<220>
   <223> synthetic peptide CCNB1 347-361
<400> 70
   Gly Ala Phe Cys Leu Ala Leu Lys Ile Leu Asp Asn Gly Glu Trp
   1 5 10 15
<210> 71
   <211> 15
   <212> PRT
   <213> artificial sequence
<220>
   <223> synthetic peptide CCNB1 351-365
<400> 71
   Leu Ala Leu Lys Ile Leu Asp Asn Gly Glu Trp Thr Pro Thr Leu
   1 5 10 15
<210> 72
   <211> 15
   <212> PRT
   <213> artificial sequence
<220>
   <223> synthetic peptide CCNB1 359-373
<400> 72
   Gly Glu Trp Thr Pro Thr Leu Gln His Tyr Leu Ser Tyr Thr Glu
   1 5 10 15
<210> 73
   <211> 15
   <212> PRT
   <213> artificial sequence
<220>
   <223> synthetic peptide CCNB1 363-377
<400> 73
   Pro Thr Leu Gln His Tyr Leu Ser Tyr Thr Glu Glu Ser Leu Leu
   1               5                   10                  15
<210> 74
   <211> 15
   <212> PRT
   <213> artificial sequence
<220>
   <223> synthetic peptide CCNB1 366-380
<400> 74
   Gln His Tyr Leu Ser Tyr Thr Glu Glu Ser Leu Leu Pro Val Met
   1               5                   10                  15
<210> 75
   <211> 15
   <212> PRT
   <213> artificial sequence
<220>
   <223> synthetic peptide CCNB1 369-383
<400> 75
   Leu Ser Tyr Thr Glu Glu Ser Leu Leu Pro Val Met Gln His Leu
   1               5                   10                  15
<210> 76
   <211> 15
   <212> PRT
   <213> artificial sequence
<220>
   <223> synthetic peptide CCNB1 374-388
<400> 76
   Glu Ser Leu Leu Pro Val Met Gln His Leu Ala Lys Asn Val Val
   1               5                   10                  15
<210> 77
   <211> 15
   <212> PRT
   <213> artificial sequence
<220>
   <223> synthetic peptide CCNB1 377-391
<400> 77
   Leu Pro Val Met Gln His Leu Ala Lys Asn Val Val Met Val Asn
   1 5 10 15
<210> 78
   <211> 15
   <212> PRT
   <213> artificial sequence
<220>
   <223> synthetic peptide CCNB1 385-399
<400> 78
   Lys Asn Val Val Met Val Asn Gln Gly Leu Thr Lys His Met Thr
   1 5 10 15
<210> 79
   <211> 15
   <212> PRT
   <213> artificial sequence
<220>
   <223> synthetic peptide CCNB1 392-406
<400> 79
   Gln Gly Leu Thr Lys His Met Thr Val Lys Asn Lys Tyr Ala Thr
   1 5 10 15
<210> 80
   <211> 15
   <212> PRT
   <213> artificial sequence
<220>
   <223> synthetic peptide CCNB1 396-410
<400> 80
   Lys His Met Thr Val Lys Asn Lys Tyr Ala Thr Ser Lys His Ala
   1 5 10 15
<210> 81
   <211> 15
   <212> PRT
   <213> artificial sequence
<220>
   <223> synthetic peptide CCNB1 402-416
<400> 81
   Asn Lys Tyr Ala Thr Ser Lys His Ala Lys Ile Ser Thr Leu Pro
   1 5 10 15
<210> 82
   <211> 15
   <212> PRT
   <213> artificial sequence
<220>
   <223> synthetic peptide CCNB1 410-424
<400> 82
   Ala Lys Ile Ser Thr Leu Pro Gln Leu Asn Ser Ala Leu Val Gln
   1 5 10 15
<210> 83
   <211> 15
   <212> PRT
   <213> artificial sequence
<220>
   <223> synthetic peptide CCNB1 416-430
<400> 83
   Pro Gln Leu Asn Ser Ala Leu Val Gln Asp Leu Ala Lys Ala Val
   1 5 10 15
<210> 84
   <211> 15
   <212> PRT
   <213> artificial sequence
<220>
   <223> synthetic peptide CCNB1 419-433
<400> 84
   Asn Ser Ala Leu Val Gln Asp Leu Ala Lys Ala Val Ala Lys Val
   1 5 10 15
<210> 85
   <211> 18
   <212> PRT
   <213> artificial sequence
<220>
   <223> synthetic peptide CCNB1 199-216
<400> 85
   Gly Asn Met Arg Ala Ile Leu Ile Asp Trp Leu Val Gln Val Gln Met
   1 5 10 15
   Lys Phe
<210> 86
   <211> 18
   <212> PRT
   <213> artificial sequence
<220>
   <223> synthetic peptide CCNB1 241-258
<400> 86
   Lys Lys Met Leu Gln Leu Val Gly Val Thr Ala Met Phe Ile Ala Ser
   1 5 10 15
   Lys Tyr
<210> 87
   <211> 20
   <212> PRT
   <213> artificial sequence
<220>
   <223> synthetic peptide CCNB1 280-299
<400> 87
   His Gln Ile Arg Gln Met Glu Met Lys Ile Leu Arg Ala Leu Asn Phe
   1 5 10 15
   Gly Leu Gly Arg
   20
<210> 88
   <211> 19
   <212> PRT
   <213> artificial sequence
<220>
   <223> synthetic peptide CCNB1 299-317
<400> 88
   Arg Pro Leu Pro Leu His Phe Leu Arg Arg Ala Ser Lys Ile Gly Glu
   1 5 10 15
   Val Asp Val
<210> 89
   <211> 18
   <212> PRT
   <213> artificial sequence
<220>
   <223> synthetic peptide CCNB1 320-337
<400> 89
   His Thr Leu Ala Lys Tyr Leu Met Glu Leu Thr Met Leu Asp Tyr Asp
   1 5 10 15
   Met Val
<210> 90
   <211> 18
   <212> PRT
   <213> artificial sequence
<220>
   <223> synthetic peptide CCNB1 363-380
<400> 90
   Pro Thr Leu Gln His Tyr Leu Ser Tyr Thr Glu Glu Ser Leu Leu Pro
   1 5 10 15
   Val Met
<210> 91
   <211> 18
   <212> PRT
   <213> artificial sequence
<220>
   <223> synthetic peptide CCNB1 416-433
<400> 91
   Pro Gln Leu Asn Ser Ala Leu Val Gln Asp Leu Ala Lys Ala Val Ala
   1 5 10 15
   Lys Val
<210> 92
   <211> 9
   <212> PRT
   <213> artificial sequence
<220>
   <223> synthetic peptide survivine
   96-104
<400> 92
   Leu Thr Leu Gly Glu Phe Leu Lys Leu
   1 5
<210> 93
   <211> 10
   <212> PRT
   <213> artificial seqeunce
<220>
   <223> synthetic peptide survivine 95-104
<400> 93
   Glu Leu Thr Leu Gly Glu Phe Leu Lys Leu
   1 5 10
<210> 94
   <211> 9
   <212> PRT
   <213> artificial sequence
<220>
   <223> synthetic peptide survivine-2B 80-88
<400> 94
   Ala Tyr Ala Cys Asn Thr Ser Thr Leu
   1 5
<210> 95
   <211> 11
   <212> PRT
   <213> artificial sequence
<220>
   <223> synthetic peptide PADRE
<220>
   <221> misc_feature
   <222> (2)..(2)
   <223> Xaa can be any naturally occurring amino acid
<400> 95
   Lys Xaa Val Ala Ala Trp Thr Leu Lys Ala Ala
   1 5 10
<210> 96
   <211> 9
   <212> PRT
   <213> artificial sequence
<220>
   <223> synthetic peptide P1
<400> 96
   Ala Gly Tyr Leu Met Glu Leu Cys Val
   1 5
<210> 97
   <211> 9
   <212> PRT
   <213> artificial sequence
<220>
   <223> synthetic peptide P2
<400> 97
   Ala Gly Tyr Leu Met Glu Leu Cys Met
   1 5
<210> 98
   <211> 9
   <212> PRT
   <213> artificial sequence
<220>
   <223> synthetic peptide P3
<400> 98
   Ala Gly Tyr Leu Met Glu Leu Cys Phe
   1 5
<210> 99
   <211> 9
   <212> PRT
   <213> artificial sequence
<220>
   <223> synthetic peptide P4
<400> 99
   Ala Gly Tyr Leu Met Glu Leu Cys Cys
   1 5
<210> 100
   <211> 10
   <212> PRT
   <213> artificial sequence
<220>
   <223> synthetic peptide P5
<400> 100
   Ala Gly Tyr Leu Met Glu Leu Cys Met Ala
   1 5 10
<210> 101
   <211> 10
   <212> PRT
   <213> artificial sequence
<220>
   <223> synthetic peptide P6
<400> 101
   Ala Gly Tyr Leu Met Glu Leu Cys Phe Ala
   1 5 10
<210> 102
   <211> 9
   <212> PRT
   <213> artificial sequence
<220>
   <223> synthetic peptide CB9
<400> 102
   Ala Lys Tyr Leu Met Glu Leu Thr Met
   1               5
<210> 103
   <211> 10
   <212> PRT
   <213> artificial sequence
<220>
   <223> synthetic peptide CB10
<400> 103
   Ala Lys Tyr Leu Met Glu Leu Thr Met Leu
   1               5                   10
<210> 104
   <211> 9
   <212> PRT
   <213> artificial sequence
<220>
   <223> synthetic peptide CB9L2
<400> 104
   Ala Leu Tyr Leu Met Glu Leu Thr Met
   1               5
<210> 105
   <211> 9
   <212> PRT
   <213> artificial sequence
<220>
   <223> synthetic peptide CB9M2
<400> 105
   Ala Met Tyr Leu Met Glu Leu Thr Met
   1               5
<210> 106
   <211> 9
   <212> PRT
   <213> artificial sequence
<220>
   <223> synthetic peptide CB204
<400> 106
   Ile Leu Ile Asp Trp Leu Val Gln Val
   1 5
<210> 107
   <211> 15
   <212> PRT
   <213> artificial sequence
<220>
   <223> synthetic peptide CB215-229
<400> 107
   Lys Phe Arg Leu Leu Gln Glu Thr Met Tyr Met Thr Val Ser Ile
   1 5 10 15
<210> 108
   <211> 15
   <212> PRT
   <213> artificial sequence
<220>
   <223> synthetic peptide CB219-233
<400> 108
   Leu Gln Glu Thr Met Tyr Met Thr Val Ser Ile Ile Asp Arg Phe
   1 5 10 15
<210> 109
   <211> 12
   <212> PRT
   <213> artificial sequence
<220>
   <223> synthetic peptide CB223-234
<400> 109
   Met Tyr Met Thr Val Ser Ile Ile Asp Arg Phe Met
   1 5 10